(19) Europäisches Patentamt
European Patent Office
Office européen des brevets

(11) EP 3 193 936 B1

(12) **FASCICULE DE BREVET EUROPEEN**

(45) Date de publication et mention de la délivrance du brevet:
**17.06.2020 Bulletin 2020/25**

(21) Numéro de dépôt: **15766125.7**

(22) Date de dépôt: **15.09.2015**

(51) Int Cl.:
*A61K 45/06* (2006.01) *A61K 31/09* (2006.01)
*A61K 31/351* (2006.01) *A61K 31/4178* (2006.01)
*A61K 31/424* (2006.01) *A61K 31/43* (2006.01)
*A61P 31/04* (2006.01) *A61P 31/10* (2006.01)
*A61K 9/00* (2006.01)

(86) Numéro de dépôt international:
**PCT/EP2015/071093**

(87) Numéro de publication internationale:
**WO 2016/041958 (24.03.2016 Gazette 2016/12)**

(54) **ANTIMICROBIENS POTENTIALISES**

POTENZIERTE ANTIMIKROBIELLE MITTEL

POTENTIATED ANTIMICROBIAL AGENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.09.2014 FR 1458663**
**09.12.2014 FR 1462136**

(43) Date de publication de la demande:
**26.07.2017 Bulletin 2017/30**

(73) Titulaire: **Septeos**
**75017 Paris (FR)**

(72) Inventeur: **TESSE, Nicolas**
**F-92420 Vaucresson (FR)**

(74) Mandataire: **Regimbeau**
**87 rue de Sèze**
**69477 Lyon Cedex 06 (FR)**

(56) Documents cités:
**US-A- 3 787 566 US-A1- 2006 134 237**

- **JEDLICKOVÁ Z ET AL: "Antibacterial properties of the Vietnamese cajeput oil and ocimum oil in combination with antibacterial agents", JOURNAL OF HYGIENE, EPIDEMIOLOGY, MICROBIOLOGY AND IMMUNOLOGY, AVICENUM, PRAGUE, CS, vol. 36, no. 3, 1 janvier 1992 (1992-01-01), pages 303-309, XP009096925, ISSN: 0022-1732**
- **HENDRY E R ET AL: "Antimicrobial efficacy of eucalyptus oil and 1,8-cineole alone and in combination with chlorhexidine digluconate against microorganisms grown in planktonic and biofilm cultures", JOURNAL OF ANTIMICROBIAL CHEMOTHERAPY, OXFORD UNIVERSITY PRESS, GB , vol. 64, no. 6 1 décembre 2009 (2009-12-01), pages 1219-1225, XP002715614, ISSN: 0305-7453, DOI: 10.1093/JAC/DKP362 Extrait de l'Internet: URL:http://jac.oxfordjournals.org/content/ 64/6/1219 [extrait le 2009-10-16] cité dans la demande**
- **Ioana Marinas ET AL: "Rosmarinus officinalis essential oil as antibiotic potentiator against Staphylococcus aureus", Biointerface Research in Applied Chemistry, 15 février 2012 (2012-02-15), pages 271-276, XP055086305, Extrait de l'Internet: URL:http://biointerfaceresearch.com/wp-content/uploads/downloads/2012/02/35.BRIAC.Grumezescu.pdf cité dans la demande**
- **Hanan H. Abd El-Kalek et al.: "SYNERGISTIC EFFECT OF CERTAIN MEDICINAL PLANTS ANDAMOXICILLIN AGAINST SOME CLINICAL ISOLATES OFMETHICILLIN-RESISTANT Staphylococcus Aureus (MRSA)", Biolt International Journal of Pharmaceutical Applications, vol. 3, no. 3 2012, pages 387-398, XP002735981, Extrait de l'Internet: URL:http://bipublication.com/files/IJPA-V3 I32012-3.pdf [extrait le 2015-02-12]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- MULYANINGSIH S ET AL: "Synergistic properties of the terpenoids aromadendrene and 1,8-cineole from the essential oil of Eucalyptus globulus against antibiotic-susceptible and antibiotic-resistant pathogens", PHYTOMEDICINE, GUSTAV FISCHER VERLAG, STUTTGART, DE, vol. 17, no. 13, 1 novembre 2010 (2010-11-01), pages 1061-1066, XP027417669, ISSN: 0944-7113 [extrait le 2010-08-19]
- GEHAN I KH MAREI ET AL: "Comparative antifungal activities and biochemical effects of monoterpenes on plant pathogenic fungi", PESTICIDE BIOCHEMISTRY AND PHYSIOLOGY, ACADEMIC PRESS, US, vol. 103, no. 1, 18 mars 2012 (2012-03-18) , pages 56-61, XP028419728, ISSN: 0048-3575, DOI: 10.1016/J.PESTBP.2012.03.004 [extrait le 2012-03-28]
- FAWAD S A ET AL: "Antimicrobial activity of Eucalyptus tereticornis and comparison with daily life antibiotics", INTERNATIONAL JOURNAL OF PHARMACEUTICAL SCIENCES REVIEW AND RESEARCH 2012 GLOBAL RESEARCH ONLINE IND, vol. 12, no. 1, janvier 2012 (2012-01), pages 21-29, XP002735982, ISSN: 0976-044X
- BACHIR RAHO G ET AL: "Antibacterial activity of the essential oils from the leaves of Eucalyptus globulus against Escherichia coli and Staphylococcus aureus.", ASIAN PACIFIC JOURNAL OF TROPICAL BIOMEDICINE SEP 2012, vol. 2, no. 9, septembre 2012 (2012-09), pages 739-742, XP002735983, ISSN: 2221-1691

## Description

L'invention a pour objet les composés de formule (I), utilisés à une dose où ils ne présentent plus de propriétés antimicrobiennes, pour leur utilisation en tant qu'agent destinés à la potentialisation des actifs antimicrobiens avec lesquels ils sont co-administrés. En particulier, la combinaison « potentialisateur(s) + antimicrobien(s) » a pour but de prévenir et/ou traiter les infections bactériennes et fongiques chez l'homme ou l'animal.

L'invention a également pour objet un procédé pour potentialiser des antimicrobiens dans lequel on co-administre avec ledit antimicrobien un composé (ou plusieurs) de formule (I). Dans ce procédé, on utilise le composé de formule (I) à une dose où il est inactif seul.

## OBJET DE L'INVENTION

### Background

L'invention a pour objet d'apporter des solutions aux problématiques liées à la baisse ou la perte d'activité des antimicrobiens durant leur période d'utilisation commerciale et médicale. L'invention est donc une solution qui s'applique aux antimicrobiens actuels et qui s'appliquera aux antimicrobiens futurs.

Après l'arrivée des antimicrobiens dans les années 1940 il est rapidement apparu que les microbes (bactéries et champignons) avaient la capacité de s'adapter aux antimicrobiens utilisés. L'efficacité de ceux-ci diminue au fil du temps et de leur utilisation. 2 stratégies existent pour lutter contre la résistance, la découverte de nouvelles molécules antimicrobiennes d'une part, et d'autre part l'association avec des molécules destinées à bloquer sélectivement les mécanismes de résistance.

Depuis une vingtaine d'années, il est observé une baisse du nombre de nouvelles molécules antimicrobiennes accédant au marché, ce qui a entrainé l'augmentation majeure de la prévalence mondiale de microbes résistants. Il s'ensuit alors une situation complexe pour les patients qui sont plus difficilement soignés de leurs infections microbiennes.

L'invention décrit l'utilisation de composés de formules (I) pour potentialiser des antimicrobiens. De façon inattendue, ces composés ont démontré leur capacité de potentialisation de l'effet des antimicrobiens à des doses faibles (de 0,01 à 100 mg/l) très éloignées de celles où ils peuvent, seuls, présenter des propriétés antimicrobiennes. En conséquence, les antimicrobiens avec lesquels les composés de formules (I) sont co-administrés présentent, du fait de la potentialisation, une activité supérieure à celle usuellement observée.

L'invention vise à potentialiser les « antimicrobiens », utilisables chez l'homme ou l'animal et non l'ensemble des composés qui présentent « des propriétés antimicrobiennes », qui eux ne sont souvent pas administrables à l'homme ou l'animal du fait d'une toxicité trop importante ou d'un seuil d'activité antimicrobienne trop élevé nécessitant des doses incompatibles avec la santé.

Il est important de rappeler que toute molécule présente, dans l'absolu, des propriétés antimicrobiennes. Les propriétés antimicrobiennes d'une molécule doivent donc s'évaluer en regard de la concentration minimale qui inhibe la bactérie.

L'invention se base sur la découverte surprenante que des composés, qui présentent des propriétés antimicrobiennes à des doses très élevées (niveau incompatible avec un usage en médecine pour cet effet) et dont les concentrations minimales inhibitrices (CMIs) supérieures à 5000 mg/L, avantageusement supérieures à 10000 mg/L, présentent des effets de potentialisation à des doses faibles (0,01 à 100 mg/l). A ces doses, il devient envisageable d'utiliser les composés (I) chez l'homme ou l'animal.

De façon encore aussi surprenante, il a été constaté que l'effet de potentialisation est meilleur à dose faible du composé (I), et que l'effet (pour les composés présentant des propriétés antimicrobiennes à dose très élevées) augmente lorsque l'on s'éloigne de la dose à laquelle le composé (I) présente seul des propriétés antimicrobiennes.

Par exemple, il a été constaté de façon inattendue que le cinéole, qui présente des propriétés antimicrobiennes à une concentration de l'ordre du g/L (CMI de 25 000mg/l à 50 000 mg/l soit 2,5 à 5%, selon les souches), présente des effets de potentialisation des antibiotiques à ces concentrations proches de la CMI (1 à 3 dilutions).

Lorsque l'on diminue sa concentration en dessous de la CMI, le cinéole ne potentialise plus les antibiotiques (voire exemples 2 et 6). Puis, de manière très surprenante, on a constaté qu'en diminuant encore significativement la concentration en cinéole, de 10 à 50 fois moins que la CMI, le cinéole présente à nouveau des effets de potentialisation sur un très grand nombre d'antibiotiques sur un très grand nombre de souches, cette fois à des doses compatibles avec l'usage médical.

Un grand avantage est que les composés (I) utilisés à ces doses éloignées de leur seuil d'efficacité, sont le plus souvent utilisables chez l'homme ou l'animal sans contraintes de toxicité du fait de l'absence de toxicité ou à des niveaux acceptables de toxicité. Cela ne serait pas le cas si l'on souhaitait les utiliser aux doses élevées où ils présentent des propriétés antimicrobiennes voire aux doses proche de leur CMI ou ils pourraient potentialiser les antimicrobiens.

De façon tout aussi surprenante l'effet de potentialisation observé avec le composé selon l'invention n'est pas

spécifique d'un mécanisme de résistance particulier mais s'observe sur diverses souches, qu'elles aient, ou n'aient pas, développé un ou plusieurs mécanismes de résistance distincts.

## ART ANTERIEUR

L'art antérieur décrit dans de nombreuses publications les propriétés antimicrobiennes de certains composés (I). Ces publications n'anticipent pas l'effet de potentialisation des composés (I) à des doses éloignées de leur seuil d'efficacité.

L'art antérieur est conséquent sur les composés (I) car ils appartiennent à des classes chimiques très étudiées dans de nombreux domaines. Les composés (I) dans le cadre de la présente invention présentent les caractéristiques suivantes qui permettent de les écarter de l'art antérieur et qui permettent d'envisager leur usage comme potentialisateur d'antimicrobiens administrés à l'homme et à l'animal:

A/ Ils sont utilisés à des doses faibles (0,01 à 100 mg/l), doses éloignées de leur seuil de propriétés antimicrobienne, leur effet de potentialisation n'est pas spécifique de mécanismes d'action ou de résistance particuliers
B/ ils ont été validés par des méthodes de screening adaptée à leurs caractéristiques physico-chimiques
C/ ils présentent, aux doses ou ils potentialisent, un ratio effet/toxicité qui permet un usage sécurisé chez l'homme ou l'animal
D/ Ils constituent une (des) entité(s) chimique(s) isolée(s) dont les caractéristiques sont reproductibles de façon constante à l'infini car leur méthode d'obtention (synthèse, hémi-synthèse, extraction) le permet.
E/ Ils ne possèdent pas de toxicité particulière qui interdirait leur usage même à dose faible (génotoxicité, cardio-toxicité,...)

A/ <u>Concernant les niveaux d'activités mesurés dans l'art antérieur</u>

Dans l'art antérieur, la concentration utilisée pour observer une activité antimicrobienne n'est pas compatible avec une utilisation future chez l'homme ou l'animal, en particulier lors d'une application systémique. Dans la majeure partie des publications scientifiques, l'usage médical est envisagé alors que les concentrations efficaces mesurées (de l'ordre de plusieurs mg/ml) sont incompatibles avec cet usage. Les effets antimicrobiens mesurés l'ont très souvent été pour des teneurs en huile essentielle, ou de son composé actif, de l'ordre de plusieurs mg/ml. Or, une telle concentration n'est pas adaptée pour une utilisation future chez l'homme ou l'animal, en particulier par voie systémique. 1 mg/ml correspond à 1 g/l ou 1g/kg ou encore 0,1%. Si la CMI était de 1 mg/ml, il faudrait, en fonction des paramètres de pharmacocinétique, administrer au moins 1 g/kg/j de poids vif. Par exemple, la dose effective pour une vache devrait être au moins 500 g/j et au moins 60 g/j pour l'homme (ceci correspond à la dose minimale car on suppose ici que le produit est totalement absorbé et distribué dans l'organisme). Ces doses bien trop importantes ne sont pas envisageables pour une utilisation sécurisée en thérapeutique.

Par exemple on peut citer deux publications qui citent l'effet antibactérien des terpénoïdes et leur éventuel capacité de potentialisation des antimicrobiens :

- Biointerfacae Vol 2, Issue 1, 2012, 271-276 : Marinas et al : Rosmarinus officinalis essential oil as antibiotic potentiator against Staphylococcus aureus. Si cette publication semble proche de l'invention il est important de noter la dose envisagée (p274) d'eucalyptol qui, à 25 µL/mL (soit 25 ml/L soit 25 g/l) se situe très loin de la dose administrable à l'homme ou l'animal. Le test de synergie envisagée est réalisé avec la solution de stockage (50% de cinéole) soit 50 g/L, supérieure à la CMI du cinéole dans la publication, dosage incompatible pour une utilisation en médecine.
- Journal of Antimicrobial Chemotherapy (2009) 64, 1219-1225 : Hendry et al : Antimicrobial efficacy of eucalyptus oil and 1,8-cineole alone and in combination with chlorhexidine digluconate against microorganisms grown in planktonic and biofilm cultures.
  Là encore il est envisagé la synergie avec un produit qui présente des propriétés antimicrobiennes. La concentration de cinéole envisagée est de 4 g/L, dose très proche de la CMI du cinéole dans la publication (8-64 g/L) mais trop importante pour être envisageable dans une administration humaine ou animale.

Clairement l'invention se différencie de ces deux publications car dans celles-ci les doses envisagées sont très élevées tandis que la concentration envisagée se situe à proximité de la CMI.

B/ <u>Concernant l'inadaptation des méthodes décrites dans l'art antérieur</u>

Les auteurs ayant travaillé selon des approches différentes (produits actifs seuls, produits naturels,...) sur les familles chimiques comprenant les composés (I) ont en général utilisé les méthodes standard de mesure d'effet antibactérien sans les adapter à la nature hydrophobe et volatile des terpénoïdes et phénylpropanoïdes.

Par exemple, WO 99/66796 (Wisconsin Alumni Research Foudation) décrit une méthode pour sensibiliser des cellules microbiennes vis-à-vis de composés antibactériens comprenant une étape de mise en contact avec un composé antibactérien et un sesquiterpenoïde, pour améliorer l'effet du composé antibactérien.

Dans cette demande, les CMI ont été déterminées avec la méthode de diffusion sur gélose, inadaptée à la nature volatile et hydrophobe des composés (I) et des familles apparentés. Cette méthode consiste à déposer des disques de papier imprégnés de quantités connues de composés à tester sur une gélose ensemencée avec la bactérie à étudier. Il s'établit sur la gélose un gradient de concentration du composé autour de chaque disque ; après 18 heures on mesure le diamètre du halo d'inhibition. Cette méthode n'est toutefois pas fiable pour les composés hydrophobes qui, en raison de tensions de surface et d'angles de contacts très différents sur les surfaces hydrophiles, interfèrent avec la formation du gradient de concentration dans la gélose. Dans certaines zones, la concentration en composés à tester est bien supérieure à la concentration théorique. Ainsi, les tests ne peuvent pas être quantitatifs, lorsqu'ils peuvent être qualitatifs. En outre, on note une dilution du composé hydrophobe à tester dans l'éthanol, sans toutefois corriger le résultat alors que l'éthanol est un composé antibactérien et volatil.

A noter que cette demande enseigne qu'aucun effet n'est obtenu avec des terpènes autres que les sesquiterpènes.

C/ Concernant la toxicité des composés de l'art antérieur

A propos des composés et compositions naturelles, il y a une confusion entre l'origine naturelle et l'absence de toxicité. Les huiles essentielles (et leurs dérivés) sont habituellement décrites comme étant peu toxiques, ce qui est souvent vrai dans des applications alimentaires ou en parfumerie mais erroné dans le cadre d'une administration thérapeutique.

A propos des composés chimiques isolés, la confusion existe aussi et repose sur l'origine naturelle (extraction) des composés.

Par exemple WO2006/120567 (Advanced Scientific developments) décrit des compositions pharmaceutiques comprenant au moins une substance thérapeutique active et décrite comme non toxique, choisie parmi carveol, thymol, eugenol, borneol, carvacrol, alpha-ionone, beta-ionone, et leurs isomères, dérivés et mélanges, et comprenant, à titre de deuxième substance thérapeutique active un antibiotique. Le carveol, thymol, eugenol, borneol, carvacrol, alpha-ionone, ou beta-ionone, utilisés seuls, présentent une activité antibactérienne et nombre d'entre eux posent cependant également des problèmes de toxicité, ignorés dans cette demande.

Par exemple, le carvacrol présente les données de toxicité suivantes: la DL 50 (souris, intraveineuse) est de 80 mg/kg tandis que la dose létale la plus faible en voie orale est de 100 mg/kg, chez deux espèces mammifères (chat et rat). Ces données sont à mettre en regard de la dose de 0,3 mg/ml (soit 300 mg/kg), envisagés dans le document.

D/ Concernant la variabilité chimique des composés décrits dans l'art antérieur

L'utilisation d'huile essentielle est problématique, à l'échelle industrielle, en termes de qualité et de reproductibilité étant donné que la composition d'une huile essentielle varie d'un lot à l'autre.

Par exemple DE 196 31 037 (Boehringer) décrit l'utilisation de l'huile essentielle d'arbre à thé pour potentialiser l'effet d'antibiotiques sur les souches Staphylococcus aureus. Le composant principal l'huile essentielle d'arbre à thé est le terpinen-1-ol.

Cette variabilité a notamment trois conséquences qui limitent industrialisation en vue d'une application chez l'homme ou l'animal:

- il est difficile d'assurer la constance de l'effet thérapeutique
- Il est difficile d'assurer la faible toxicité des produits
- le coût relatif à l'approvisionnement et au management de la qualité et de la reproductibilité des matières est important.

Le tableau suivant récapitule l'enseignement de ces arts antérieurs :

Tableau 1

| | A/ Produits actifs à dose envisagée | B/ Méthode de screening incompatible avec la nature chimique des composés | C/ Toxicité à la dose envisagée | D/ Problème de variabilité chimique | Dose envisagée |
|---|---|---|---|---|---|
| Marinas et al | Oui | Oui | Oui | Non | 15 g/L soit 15 000 mg/l |
| Hendry et al | Oui | Non | Oui | Non | 4 g/L soit 4 000 mg/l |
| WO 2009/043987 Aromatechnologies | Oui | Oui | Oui | Oui | 0,1 à 0,4 % soit: 1 à 4 000 mg/L |
| WO2006/120567 Advanced | Oui | Oui, (pas d'agent dispersant) | Oui | Non | 0,3 mg/ml soit 3 000 mg/l |
| DE 19631037 Bœhringer | Oui | Non (test dans le lait) | Oui | Oui | 1 à 2 mg/ml soit 1000 à 2000 mg/l |
| WO 99/66796 Wisconsin Alumni | Oui | Oui | Oui | Non | 1 mM soit 222 mg/L (pour le sesquiterpenoides) |
| Présente Invention | Non | Non | Non | Non | 0,01 à 100 mg/L |

Le travail portant sur des composés (I) à des doses faibles éloignées de celles auxquelles ils présentent une activité antimicrobienne est nouveau et l'art antérieur n'a pas, à notre connaissance, envisagé cet usage.

**DEFINITIONS**

Par « micro-organisme », on entend tout organisme vivant, invisible à l'œil nu en raison de ses faibles dimensions.

Par « organisme », on entend toute entité biologique (être vivant) animale ou végétale capable de naître, de se développer et normalement de se reproduire.

Dans ce brevet la définition de microbe reprend la définition de micro-organisme, limitée au champ médical auquel l'invention se réfère. Ainsi les « microbes » sont les microorganismes vivants potentiellement pathogènes (bactéries, champignons, levure et mycobactéries). Le terme exclue donc les pathogènes inertes comme les virus et les prions.

Par « antimicrobien » on entend tout composé destiné à être administré à l'homme ou l'animal capable de tuer ou d'inhiber la croissance de microbes. Les sels pharmaceutiquement acceptables de ces antimicrobiens sont également inclus dans cette définition. Cela inclut, par exemple, les sels de sodium, de potassium, de calcium, etc. et les sels aminés de procaine, dibenzylamine, éthylendiamine, éthanolamine, méthylglucamine taurine, etc., de même que les sels d'addition acide tels que les hydrochlorures et les acides aminés basiques. Le terme regroupe ainsi les antibiotiques (leurs associations avec les inhibiteurs de mécanismes de résistance), les antifongiques destinés à un usage systémique ou local.

Par « propriétés antimicrobiennes » on entend les propriétés d'une quelconque substance capable de détruire ou d'inhiber la croissance des microbes. Les produits qui présentent des propriétés antimicrobiennes comprennent notamment les antimicrobiens et les biocides.

Par opposition au terme « antimicrobien » qui regroupe les antibactériens, antifongiques destinés à être administrés, le terme « biocide » regroupe les produits ayant des propriétés antimicrobiennes destinés à être appliqués à des systèmes inertes (virus et prions).

Par « propriétés antibactériennes » et « propriétés antifongiques » on entend non seulement les propriétés bactéricides et fongicides caractérisées par la destruction des bactéries et champignons (et levures, mycobactéries), mais également les propriétés bactériostatiques et fongistatiques, caractérisées par l'inhibition de la croissance desdites bactéries et champignons (et levures, mycobactéries). Les produits qui présentent des propriétés antibactériennes ou antifongiques comprennent notamment les antimicrobiens.

Par « bactérie résistante » aux antibiotiques on entend, au sens de la présente invention, une bactérie résistante à au moins un, notamment au moins deux, en particulier au moins trois, voire au moins quatre, antibiotique(s) ou famille(s) d'antibiotique, classiquement utilisé(s).

Par « bactérie multi-résistante » on entend, au sens de la présente invention une bactérie résistante à plusieurs

antibiotiques, en particulier pour lesquels la souche devrait être sensible, ou a priori sensible, tout particulièrement une bactérie qui présente au moins deux résistances non naturelles.

On distingue les « résistances naturelles » des « résistances acquises ». Certains antibiotiques n'ont jamais été efficaces, à des doses non toxiques, contre certaines souches ou espèces bactériennes. Il s'agit d'une résistance naturelle. Lorsque des antibiotiques normalement efficaces ne s'avèrent pas ou peu efficaces vis-à-vis d'une bactérie, cette bactérie a développé une résistance acquise.

Une « infection microbienne » au sens de la présente invention désigne une infection provoquée par une ou plusieurs souches microbiennes et inclut les phases allant de la colonisation de l'hôte aux phases pathologiques. L'expression « infection microbienne » englobe donc tout effet néfaste, signe clinique, symptôme ou toute maladie apparaissant chez l'homme ou l'animal suite à la colonisation par le microbe.

Par "terpenoïde" on entend selon l'invention tout composé comprenant un squelette proche d'un terpène. Un « terpène » désigne un dérivé de l'isoprène qui est obtenu par voie biologique par la condensation d'unités en C5, conduisant par exemple aux monoterpènes, sesquiterpenes. Par « proche » on entend que le squelette est similaire à un terpène ou différent en ce qu'au moins un substituant alkyle, normalement présent, peut être absent ou porté par un autre atome. Le squelette peut en outre être substitué par des radicaux variés tels que des radicaux aliphatiques, saturés ou insaturés, linéaires ou cycliques (alkyles, alcényles, alkylènes), oxy, des aldéhydes, des esters, des alcools, des éthers et leurs équivalents soufrés ou azotés. Le terpénoïde peut avantageusement être d'origine naturelle.

Par "phenylpropanoide" on entend selon l'invention tout composé comprenant un squelette proche d'un phénylpropane. Un « phenylpropane » désigne un dérivé obtenu par synthèse biologique à partir du phénylpropane et conduisant à des dérivés en C6 (aromatique)-C3 (aliphatique) ou en C6 (aromatique)-C1 (aliphatique) et aux lactones correspondantes. Par « proche » on entend que le squelette est similaire à un phénylpropane, en particulier on retrouve le motif phényle, ou différent en ce qu'au moins un substituant alkyle, normalement présent, peut être absent ou porté par un autre atome. Le squelette peut en outre être substitué par des radicaux variés tels que des radicaux aliphatiques, saturés ou insaturés, linéaires ou cycliques (alkyles, alcényles, alkylènes), oxy, des aldéhydes, des esters, des alcools, des éthers et leurs équivalents soufrés ou azotés. Le phénylpropanoïde peut avantageusement être d'origine naturelle.

Le terme « prophylaxie » ou « prévenir une infection » tel qu'utilisé dans la présente demande désigne tout degré de retardement dans le moment d'apparition de signes cliniques ou de symptômes de l'infection, ainsi que tout degré d'inhibition de la sévérité des signes cliniques ou symptômes de l'infection, y compris mais sans limitation à, la prévention totale de ladite infection. Ceci nécessite que l'antimicrobien et le composé selon l'invention soient co-administrés à l'homme ou l'animal susceptible d'être colonisé par une souche microbienne à titre préventif, par exemple suite à un acte de chirurgie, d'implantation d'un dispositif médical, un acte médical intrusif. Cette administration prophylactique peut avoir lieu avant, pendant ou après l'acte susceptible de provoquer une infection (en particulier une infection nosocomiale) dans le but d'empêcher, améliorer, et/ou réduire la sévérité de n'importe quelle infection subséquente.

Le terme « traitement » au sens de la présente demande implique que l'antimicrobien et le composé selon l'invention soient co-administrées à un sujet (humain ou animal) au moment de la colonisation ou après la contamination ou la suspicion de contamination par une souche microbienne susceptible de provoquer une infection telle qu'une infection nosocomiale. Le terme « traitement » ou « traiter une infection » inclut donc :

- tout effet curatif (inhibition de la croissance ou destruction du microbe) obtenu grâce à la co-administration antimicrobien + composé selon l'invention ainsi que l'amélioration des signes cliniques ou des symptômes observés de même que l'amélioration de l'état du sujet.
- le ralentissement, l'interruption, ainsi que l'arrêt de la progression de l'infection. La co-administration antimicrobien-composé selon l'invention peut en effet également permettre de ralentir la progression d'un microbe et/ou empêcher complètement ou partiellement une infection microbienne de s'étendre aux tissus environnants et au-delà.
- l'inhibition, l'atténuation ou la prévention des conséquences néfastes de l'infection telles que les dommages cellulaire ou physiologiques provoqués par les toxines produites par certains microbes au niveau des tissus infectés ou avoisinants.

Le terme « co-administrés » signifie que l'antimicrobien (ou le mélange antimicrobien) et le composé selon l'invention (ou le mélange de composés selon l'invention) sont administrés sous forme combinée ou juxtaposée au sujet (homme ou animal). La combinaison inclut toute association médicamenteuse, toute composition pharmaceutique, tout kit pharmaceutique, et tout médicament comprenant (i) au moins un antimicrobien et (ii) au moins un composé selon l'invention. Les composés (i) et (ii) peuvent être présents sous la forme d'un mélange ou sous la forme de formulations ou compositions distinctes dans ladite combinaison. La combinaison peut également comprendre plusieurs antimicrobiens, par exemple 2, 3 ou 4 antimicrobiens ou plus, et/ou plusieurs composés selon l'invention, en particulier 2 ou 3 ou plus composés selon l'invention. Ces constituants forment une unité fonctionnelle du fait d'une indication commune, qui est la mise en œuvre d'un traitement antimicrobien. Cette thérapie combinée est plus spécifiquement destinée à la prophylaxie et/ou au traitement d'infections et de maladies d'origine microbienne, en particulier des infections nosocomiales.

La co-administration peut être simultanée ou étalée dans le temps.

Le terme « simultané » signifie que l'antimicrobien (ou le mélange antimicrobien) et le composé selon l'invention (ou le mélange de composés selon l'invention) sont administrés en même temps, au même moment, à un sujet (homme ou animal). Ces composés peuvent être administrés sous la forme d'un mélange ou, simultanément mais séparément, sous la forme de compositions distinctes.

L'expression « administration séquentielle » signifie que l'antimicrobien (ou le mélange antimicrobien) et le composé selon l'invention (ou le mélange de composés selon l'invention) sont administrés non pas simultanément mais séparément dans le temps, l'un après l'autre.

L'expression « pontentialiser » un antimicrobien signifie que l' utilisation d' un composé selon l'invention permet d'obtenir un effet prophylactique ou thérapeutique supérieur à l'effet prophylactique ou thérapeutique obtenu en utilisant le ou lesdits antimicrobiens seuls. Ceci peut s'exprimer de différentes manières, alternatives ou cumulatives : augmentation de l'effet de l'antimicrobien, diminution de la dose d'antimicrobien à effet constant d'antimicrobien, réduction de la CMI. En outre, la potentialisation permet de réduire, voire annihiler, l'apparition de résistance.

L'expression augmenter l'effet d'un antimicrobien signifie : l'élargissement du spectre microbien de l'activité de l'antimicrobien, l'augmentation de la vitesse d'action de l'antimicrobien, l'amélioration du succès clinique (cure rate) ou de la vitesse de l'obtention du succès clinique (time to cure) de l'antimicrobien, à dose constante d'antimicrobien.

L'expression « diminuer la quantité d'antimicrobien utilisée » signifie que l'utilisation de composé selon l'invention permet d'utiliser une quantité d'antimicrobien inférieure à la quantité d'antimicrobien normalement nécessaire pour obtenir un effet thérapeutique ou prophylactique donné lorsque l'antimicrobien est administré seul. La diminution de la quantité d'antimicrobien utilisée peut être plus ou moins importante ; elle est de préférence d'au moins 10%, et plus préférablement d'au moins 20 %, encore plus préférablement d'au moins 40%, voire 50% ou plus par rapport à la quantité normalement nécessaire pour obtenir un effet thérapeutique ou prophylactique donné.

« CMI » signifie « concentration minimale inhibitrice », qui est la plus faible concentration de substance à laquelle on n'observe plus de croissance microbienne après 18 à 24h de contact dans des conditions favorables à la croissance microbienne

Les tests de mesure de concentration minimale inhibitrice sont réalisés en milieu solide gélosé selon les normes internationales en vigueur (normes CLSI M7-A9 Jan 12) : dispersion des composés à tester dans une gélose Mueller Hinton gelose. Une adaptation relative à l'hydrophobicité des composés et compositions est cependant nécessaire pour les disperser dans le milieu : dilution des composés dans un solvant. Les composés et compositions incorporés dans la gélose peuvent être au préalable dilués dans un ou plusieurs solvant (Tween® 80 dilué dans l'eau, tween® 80 dilué dans le propylène, DMSO dilué dans l'eau). Les souches sont déposées sur la surface de la gélose avec un appareil de steers. Dans les exemples, différentes méthodes de dissolution des produits sont testées en parallèle pour contourner les problématiques de coefficient de répartition eau/solvant des molécules (antimicrobien et potentialisateur) tandis que les bactéries poussent dans la seule phase aqueuse. La contrainte technique n'interviendra pas dans les tests *in vivo.* Les mêmes méthodes de dilutions peuvent être mise en œuvre en milieu liquide (microplaques et tubes). La même méthodologie est mise en œuvre avec les champignons.

Les CMI 50 et CMI 90 représentent respectivement les concentrations qui inhibent 50% et 90% en nombre des souches d'un même genre.

Par « atome d'halogène », on entend, au sens de la présente invention, les atomes de fluor, de chlore, de brome et d'iode.

Par « hétéroatome », on entend, au sens de la présente invention, N, O ou S, avantageusement O.

Par groupement « ($C_1$-$C_6$)alkyle », on entend, au sens de la présente invention, une chaîne hydrocarbonée monovalente saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « ($C_2$-$C_6$)alcényle », on entend, au sens de la présente invention, une chaîne hydrocarbonée monovalente, linéaire ou ramifiée, comportant au moins une double liaison et comportant 2 à 6 atomes de carbone. A titre d'exemple, on peut citer les groupes éthényle ou allyle.

Par « ($C_1$-$C_6$)halogénoalkyle », on entend, au sens de la présente invention, un groupe ($C_1$-$C_6$)alkyle, tel que défini ci-dessus, pour lequel un ou plusieurs atomes d'hydrogène ont été remplacés par un atome d'halogène tel que défini ci-dessus. Il peut s'agir en particulier d'un groupe CF3.

Par groupement « ($C_1$-$C_6$)alcoxy », on entend, au sens de la présente invention, un groupe ($C_1$-$C_6$)alkyle tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore tert-butoxy.

Par groupement « ($C_2$-$C_6$)alcénoxy », on entend, au sens de la présente invention, un groupe ($C_2$-$C_6$)alcényle, tel que défini ci-dessus, lié au reste de la molécule par l'intermédiaire d'un atome d'oxygène. A titre d'exemple, on peut citer le groupe $-OCH_2CH=CH_2$.

Par groupement « ($C_1$-$C_6$)alkylène » ou « ($C_1$-$C_6$)alcanediyle », on entend, au sens de la présente invention, une chaîne hydrocarbonée divalente, linéaire ou ramifiée, comprenant 1 à 6 atomes de carbone, comme par exemple, un groupement methylène, éthylène, propylène, butylène, pentylène ou encore hexylène.

Par groupement « ($C_2$-$C_6$)alcénylène » ou « ($C_2$-$C_6$)alcènediyle », on entend, au sens de la présente invention, une chaîne hydrocarbonée divalente, linéaire ou ramifiée, comprenant 2 à 6 atomes de carbone et au moins une double liaison, comme par exemple, un groupement vinylène (éthénylène) ou propénylène.

**DESCRIPTION DE L'INVENTION**

L'invention a pour objet un agent antimicrobien potentialisé par un composé de formule I pour son utilisation dans le traitement d'une infection microbienne. Elle a ainsi pour objet une association d'un agent antimicrobien et d'un composé répondant à la formule I suivante :

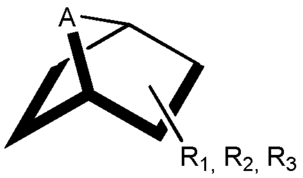
(I)

dans laquelle $R_1$, $R_2$, $R_3$ représentent chacun, indépendamment l'un de l'autre, H, OH un groupe ($C_1$-$C_6$)alkyle, ($C_2$-$C_6$)alcényle, ($C_1$-$C_6$)halogénoalkyle, ($C_1$-$C_6$)alcoxy, ($C_2$-$C_6$)alcénoxy, ($C_1$-$C_6$)halogénoalcoxy, -O-CO-($C_1$-$C_6$)alkyle ;

A représente un hétéroatome ou un groupe R-(Het)-R' ou -(Het)-R où R, R', représentent chacun, indépendamment l'un de l'autre, un groupe ($C_1$-$C_4$)alcanediyle éventuellement substitué par des radicaux alkyles en $C_1$-$C_4$ et Het représente un hétéroatome, A représente avantageusement un groupe -(Het)-R

en un ratio massique, composé de formule (I) : agent antimicrobien, variant de 8 :1 à 1 :10, en ce que l'agent antimicrobien n'est pas un terpénoïde ou un phenylpropanoide et en ce que ledit composé de formula (I) est adapté pour une administration par voie systémique.

L'agent antimicrobien est avantageusement un actif de la médecine conventionnelle occidentale, tel que cela sera décrit par la suite. En particulier, l'agent antimicrobien n'est pas un terpénoïde ou un phenylpropanoide, tel qu'un extrait d'huile essentielle ou un composant d'huile essentielle.

Le ratio massique composé de formule (I) : agent antimicrobien variant plus avantageusement de 4 :1 à 1 :10, plus avantageusement de 1 : 1 à 1 :10, encore plus avantageusement de 1 :1 à 1 :5.

Cela signifie que la dose administrée en composé de formule (I) est du même ordre de grandeur, que celui de l'agent antimicrobien.

Le ratio massique correspond au ratio des doses en mg/kg du composé et de l'agent antimicrobien à administrer à l'homme ou l'animal.

D'une manière surprenante, il a été constaté que l'effet de potentialisation diminue lorsque la dose en composé selon l'invention (ou mélange de composés selon l'invention) augmente. Cet effet de potentialisation peut réapparaître à la CMI du composé seul, mais cela n'est pas l'objet de l'invention.

Dans le cadre de la présente invention, *in vitro,* la concentration en composé inactif, à laquelle on observe un effet de potentialisation, est très éloignée du seuil des propriétés antimicrobiennes (CMI), lorsque des propriétés antimicrobiennes sont observées. Par très éloignée, on entend que la concentration *in vitro* est au moins 10 fois, avantageusement au moins 20 fois, plus avantageusement au moins 50 fois, encore plus avantageusement au moins 100 fois inférieure à la CMI.

En particulier, sur une souche A, pathogène qui induit notamment l'infection microbienne considérée, la concentration en composé (I) répond à l'équation suivante :

$$[C] < [CMI] / x$$

Où [C] est la concentration selon l'invention en composé (I) à utiliser sur la souche A

[CMI] est la CMI mesurée pour le composé (I), seul, sur cette souche A

x est supérieur ou égal à 100, avantageusement à 1 000, plus avantageusement x est compris entre 2 000 et 10 000, voire supérieur à 50 000.

*In vitro,* les doses en composé (I) sont inférieures à 100 mg/L, avantageusement inférieures à 64 mg/L, plus avantageusement comprises entre 0,01 et 25 mg/L, encore plus avantageusement comprises entre 1 et 16 mg/L.

Dans les compositions administrées, la concentration, par unité de dose par kilo, en composé de formule (I) est avantageusement inférieure à 100 mg, plus avantageusement inférieure à 64mg.

Les composés (I) peuvent être utilisés à une concentration faible (*in vitro* à des concentrations de l'ordre du μg/ml) pour potentialiser les antimicrobiens, ce qui est tout à fait compatible avec une utilisation future chez l'homme ou l'animal (en particulier lorsqu'une administration systémique est recherchée).

Cela permet d'envisager des dosages chez l'homme ou l'animal inférieurs à 64 mg/kg, avantageusement compris entre 0,01 et 64 mg/kg, plus avantageusement compris entre 0,5 et 40 mg/kg, encore plus avantageusement compris entre 5 et 30 mg/kg.

Le composé potentialisateur selon l'invention est avantageusement administré à une concentration telle que sa concentration sérique maximale soit inférieure à 250 mg/L, avantageusement inférieure à 150 mg/L, plus avantageusement comprise entre 10 et 150 mg/L après administration.

Bien entendu, à ces concentrations, le composé peut être administré à l'homme et l'animal, y compris par voie systémique, et ne présente pas d'effets indésirables majeurs, en particulier de carcinogénicité ou de génotoxicité.

Le ratio massique composé selon l'invention (ou mélange de composés selon l'invention) :antimicrobien dépend à chaque fois de l'antimicrobien utilisé et il sera adapté au cas par cas.

Par exemple, pour l'amoxicilline dans les cas où elle est usuellement administrée à une dose de 1000 mg par prise, la dose en composé de formule (I) pourra varier entre 300 et 850 mg par prise, voire moins. Par contre, pour la colistine qui est usuellement administrée à une dose de 1 MUI par voie pulmonaire, la dose en composé de formule (I) pourra varier entre 0,1 et 0,8 MUI, voire moins.

Autrement formulée, l'invention a pour objet un procédé pour potentialiser l'activité antimicrobienne d'un antimicrobien de façon indépendante du mécanisme de résistance comprenant les étapes suivantes :

a) Choisir un composé de formule (I) qui soit thérapeutiquement inactif (à visée anti-infectieuse) seul à la dose envisagée,

b) Préparer une composition comprenant le composé choisi à l'étape a) avec l'antimicrobien

Le composé et l'antimicrobien sont adaptés pour une administration simultanée, séparée ou étalée dans le temps à l'homme ou l'animal.

L'antimicrobien est de préférence un antibiotique. Il peut aussi être un antifongique.

D'une manière surprenante, il a été constaté que les composés de formule (I), utilisés à cette faible concentration, sont capables de potentialiser l'activité des antimicrobiens. Ainsi, l'utilisation de ces potentialisateurs permet avantageusement d'utiliser ledit antimicrobien à une concentration plus faible et/ou, à concentration usuelle tout en ayant une activité supérieure que l'antimicrobien seul à la même dose (augmentation de l'intensité de l'effet ou de la cinétique de l'effet).

Concrètement, l'invention permet notamment:

A/ diminuer les doses à effet constant : diminution la quantité nécessaire d'un antimicrobien pour inhiber/détruire les microbes habituellement sensibles

B/ d'augmenter l'effet à dose constante : augmentation de la capacité d'un antimicrobien à inhiber/détruire les germes sensibles (amélioration de la cinétique de l'effet, de l'intensité de l'effet, et élargissement du spectre d'activité d'un antimicrobien vers des germes qui étaient inconstamment sensibles ou résistant à l'antimicrobien).

Réduire la dose administrée (A/) d'un antimicrobien présente un intérêt non seulement du point de vue du traitement des infections microbiennes chez l'homme ou l'animal notamment la réduction des effets secondaires, mais également, et ceci n'est pas négligeable, d'un point de vue environnemental (diminution de l'apparition de résistances aux d'antimicrobiens). L'utilisation des antimicrobiens connus à des doses plus faibles peut aider dans la lutte contre l'apparition de nouveaux mécanismes de résistance. En particulier, l'antimicrobien peut être utilisé à une dose réduite, dans laquelle la dose administrée en antimicrobien correspond de 1/50 à 3/4 de la dose nécessaire en antimicrobien en absence de la co-administration d'un composé selon l'invention pour une administration à un sujet (homme, animal) pour traiter les infections microbiennes. La diminution de la dose d'antimicrobien à effet constant permet de limiter la toxicité dudit antimicrobien. En application chez l'animal de rente, cela permet de diminuer les temps de latence avant abattage.

La réduction de la dose permet également d'envisager la ré-utilisation de certains antimicrobiens, qui à ce jour ne peuvent plus être administrés parce qu'ils présentent à leurs doses efficaces des effets secondaires importants, et peuvent de nouveau être administrés efficacement chez l'homme ou l'animal avec peu d'effets secondaires.

Augmenter l'effet d'un antimicrobien à dose constante (B/) présente un intérêt clinique certain tant du point de

vue quantitatif en améliorant la cinétique d'un effet antimicrobien que qualitatif en rendant possible de traiter un patient (humain ou animal) souffrant d'une infection microbienne avec un antimicrobien pour lequel la souche était sensible ou inconstamment sensible en l'absence de potentialisation. L'augmentation de la vitesse de l'effet de l'antimicrobien permet de diminuer le temps passé à l'état « infectant » par le patient ou l'animal, réduisant ainsi l'épidémiologie de la maladie ainsi que l'apparition et la diffusion des résistances.

Grâce à la présence des composés selon l'invention, il est possible d'augmenter la vitesse de bactéricidie de l'antimicrobien à dose constante d'antimicrobien. Ainsi, la vitesse d'action d'un antimicrobien potentialisé peut être augmentée. Ceci est particulièrement vrai pour des antimicrobiens concentration-dépendant.

Grâce à la présence des composés selon l'invention, le spectre de l'antimicrobien peut être élargi, notamment à dose constante d'antimicrobien. Ainsi, un antimicrobien potentialisé par les composés selon l'invention peut être utilisé sur des souches sur lesquelles il n'est plus sensible en l'absence de potentialisation (notamment en raison de l'apparition de résistances).

Dans un mode de réalisation, le composé de formule (I) est suffisant pour potentialiser l'antimicrobien, avec pour conséquence que l'utilisation d'un seul composé de formule (I) est suffisant pour potentialiser l'antimicrobien. Cependant, dans certains cas, une utilisation combinée de composés inactifs peut être envisagée. Cela est particulièrement vrai lorsque qu'une activité large spectre est recherchée. En particulier, pour potentialiser un antibiotique sur les bactéries à Gram + et à Gram -, il peut être utile de co-administrer un composé selon l'invention particulièrement potentialisateur sur les bactéries à Gram + et un composé selon l'invention particulièrement potentialisateur sur les bactéries à Gram -.

Dans la formule (I) $R_2$ et $R_3$ représentent chacun H, $R_1$ représente un groupe $(C_1-C_6)$alkyle, et A représente l'hétéroatome O ou un groupe R-O-R' ou -O-R', où R, R', représentent chacun, indépendamment l'un de l'autre, un groupe $(C_1-C_2)$alcanediyle, éventuellement substitué par des radicaux alkyles en $C_1-C_4$.
Avantageusement le composé de formule (I) est le cinéole.

Dans le cadre de la présente invention, l'infection microbienne est avantageusement une infection induite par un pathogène choisi parmi les genres potentiellement pathogènes suivants: *Acetobacter, Acetobacterium, Acinetobacter, Citrobacter, Enterobacter, Enterococcus, Escherichia, Helicobacter, Klebsiella, Proteus, Providencia, Pseudomonas, Salmonella, Serratia*, *Staphylococcus, Streptococcus, Actinobacillus, Neisseria, Mannheima, Pasteurella, Candida, Aspergillus, Cryptococcus, Trichosporon, Malassezia,* et *Mycobacterium.*

La souche ou espèce bactérienne est avantageusement choisie dans le groupe constitué de: *Acetobacter, Acetobacterium, Acinetobacter, Actinobacillus, Citrobacter, Enterobacter, Enterococcus, Escherichia, Helicobacter, Klebsiella, Mannheima, Pasteurella, Proteus, Providencia, Pseudomonas, Salmonella, Serratia, Staphylococcus,* et *Streptococcus.* Plus particulièrement, la souche ou espèce bactérienne est avantageusement choisie dans le groupe constitué de *Citrobacter freundii, Enterobacter aerogenes, Enterobacter cloacae, Escherichia coli, Klebsiella oxytoca, Klebsiella pneumoniae, Proteus mirabilis, Providencia stuartii, Salmonella sp, Serratia marcescens, Acinetobacter baumannii, Burkholderia cepacia, Pseudomonas aeruginosa, Staphylococcaceae, Staphylococcus aureus, Enterococcus faecium,* et *Enterococcus sp.* Ainsi, la bactérie peut indifféremment être une bactérie à gram - ou une bactérie à gram +.

La bactérie est plus avantageusement choisie dans le groupe constitué de *Pseudomonas aeruginosa, Escherichia coli, Enterococcus faecalis, Klebsiella pneumoniae et Staphylococcus aureus.*

Le champignon est avantageusement choisi dans le groupe constitué de: *Candida, Aspergillus, Cryptococcus, Trichosporus.* Le champignon est plus avantageusement candida albicans.

La mycobactérie est avantageusement le *Mycobactérium tuberculosis*

L'agent antimicrobien peut être un antibiotique et/ou un agent antifongique. Dans une variante préférée, le composé de formule (I) et l'antimicrobien n'appartiennent pas à la même famille de composés chimiques. Ainsi, le composé de formule (I) étant dérivé d'un terpenoïde, l'antimicrobien n'est pas un terpenoïde, voire même n'est pas un extrait d'huile essentielle ou un phénylpropanoïde.

Les antibiotiques, qui peuvent être utilisés dans la présente invention sont avantageusement choisis parmi :

1. Les antibiotiques actifs sur les membranes, en particulier les bêtalactamines, les pénicillines, les céphalosporines, les glycopeptides, la fosfomycine, les polymixines, la bacitracine, la cyclosérine ;
2. Les antibiotiques inhibant la synthèse des protéines, en particulier les aminosides, des téracyclines, l'acide fusidique, le chloramphenicol et ses dérivés, les macrolides, les lincosamides, les streptogramines, les synergistines et oxazolidinones;
3. Les antibiotiques inhibant la synthèse des acides nucléiques, en particulier les quinolones, les nitrofuranes, les ansamycines et l'acide fucidique ;
4. Les antibiotiques inhibant la synthèse des folates, en particulier les sulfamides et associations sulfamidées ;
5. Les antibiotiques inhibant la synthèse des acides mycoliques, en particulier l'isoniazide, la prothionamide, l'éthionamuide, la pyrazynamide
6. L'un quelconque de leurs sel pharmaceutiquement acceptables, et

7. L'une quelconque de leurs combinaisons.

De préférence, l'antibiotique est choisi parmi : les antibiotiques inhibant la synthèse du peptidoglycane, les antibiotiques inhibant la synthèse des acides nucléiques, les antibiotiques inhibant la synthèse des folates, les antibiotiques inhibant la synthèse des acides mycoliques, l'un quelconque de leurs sel pharmaceutiquement acceptables, et l'une quelconque de leurs combinaisons. La classe d'antibiotique particulièrement avantageuse est celle des antibiotiques inhibant la synthèse du peptidoglycane.
En particulier, l'antibiotique est choisi parmi: amoxicilline, amoxicilline / acide clavulanique, imipenem, vancomycine, erythromycine, azithromycine, gentamicine, amikacine, colistine, clindamycine, ciprofloxacine, tigecycline.
Dans une variante de l'invention, l'infection microbienne est une infection bactérienne induite par une souche bactérienne, de type cocci ou bacilles à gram positif, et l'antimicrobien est l'amoxicilline.
Dans une variante de l'invention, l'amoxicilline est dosée à 1000 mg/prise et le cinéole est dosé à 1000mg / prise, avantageusement 500 mg/prise encore plus avantageusement à 250 mg/prise.
Dans une autre variante de l'invention, l'infection microbienne est une infection bactérienne induite par une souche bactérienne de type *E. coli* ou *S. aureus* résistant à la methicilline et l'antimicrobien est un mélange amoxicilline/ acide clavulanique.
Dans une autre variante de l'invention le ratio mélange amoxicilline/ acide clavulanique : cinéole est de 10 : 1, 1 : 1 voire 1 :5
Dans une autre variante de l'invention, l'infection microbienne est une infection bactérienne induite par une souche de type entérobactérie, *Pseudomonas* ou *S. aureus* et l'antimicrobien est la ciprofloxacine.
Les antifongiques, qui peuvent être utilisés dans la présente invention sont avantageusement choisis parmi :

1. Les antifongiques agissant sur la membrane, en particulier les polyènes, les azolés les allylamines et thiocarbamates, les echinocandines
2. Les antifongiques agissant sur la synthèse des acides nucléiques, en particulier la griseofulvine, la fluorocytosine
3. Les antifongiques agissant sur les microtubules, en particulier la griseoful vine

L'antifongique est avantageusement choisi parmi les polyènes, les azolés, les allylamines, les thiocarbamates, les echinocandines, la griseofulvine, et la fluorocytosine.
Dans une autre variante de l'invention, l'infection microbienne est une mycose vaginale et l'antibiotique est le sertaconozole.
Les composés selon l'invention sont administrés par voie systémique. Ils sont ainsi adaptés pour une administration par voie systémique.
Les composés selon l'invention peuvent être utilisés dans toute composition pharmaceutique formulée de façon à faciliter son administration. La composition pharmaceutique peut comprendre tous les excipients pharmaceutiquement acceptables habituellement utilisés tels que des véhicule(s) ou diluant(s).
La composition pharmaceutique peut être administrée par voie orale, entérale, parentérale (intraveineuse, intramusculaire ou sous-cutanée, intraperitoneale), transcutanée (ou transdermique ou percutanée), cutanée, transmuqueuse-buccale, nasale, vaginale, rectale, ou encore par voie intragastrique, intracardiaque, intrapéritonéale, intrapulmonaire ou intratrachéale.
La composition pharmaceutique peut se présenter sous forme sèche, forme sèche à reconstituer au moment de l'utilisation (poudre, lyophilisât, etc.), solide (en particulier cachet, poudre, gélule, pilule, granule, suppositoire, comprimé, et plus précisément comprimé à libération accélérée, comprimé gastrorésistants ou comprimé à libération prolongée), pâteuse (en particulier gel, pommade, crème ou ovule), liquide (en particulier sirop, solution injectable, infusible ou buvable ou collyre), sous forme d'un aérosol (spray, vapeur ou gaz), sous la forme d'un patch, sous forme injectable (dans une solution aqueuse, non aqueuse ou isotonique)
Par ailleurs, la composition pharmaceutique peut être conditionnée pour une administration sous la forme d'une dose unique (monodose) ou multiple (multidose).
Le ou les antimicrobien (s) et le ou les composé(s) selon l'invention peuvent être administrés dans une même composition pharmaceutique ou dans des compositions pharmaceutiques distinctes, de manière simultanée, séquentielle ou étalée dans le temps. En cas d'administration séparée, les formes des compositions pharmaceutiques peuvent être similaires ou distinctes ; les voies d'administration pouvant être identiques ou distinctes.
Le schéma d'administration sera adapté par le praticien en fonction des cas. Les voies d'administration et les posologies varient en fonction d'une variété de paramètres, par exemple en fonction de l'état du patient, du type d'infection et de la sévérité de l'infection à traiter ou de l'antimicrobien utilisé.
L'animal est de préférence un mammifère, en particulier l'homme, les animaux de compagnie, les animaux de rente.
Les exemples qui suivent illustrent l'invention.

Mesure de CMI : Les tests de mesure de concentration minimale inhibitrice sont réalisés en milieu solide gélosé selon les normes internationales en vigueur (normes CLSI), selon le protocole défini précédemment. Les composés et compositions incorporés dans la gélose peuvent être au préalable dilués dans un ou plusieurs solvant (Tween® 80 dilué dans l'eau -3,4 ml de Tween pour 9,6 ml d'eau- , tween® 80 dilué dans le propylène 10 - 3,4 ml de Tween pour 9,6 ml de propylène-, ou le DMSO dilué dans l'eau).

L'apport du booster comparé à la CMI de l'antibiotique seul est exprimé en ratio (CMI ATB)/ (CMI ATB + booster). On définit ainsi des gains :

Gain CMI 50= (CMI50 de l'antibiotique seul) / (CMI50 (antibiotique+booster))
Gain CMI 90= (CMI90 de l'antibiotique seul) / (CMI90 (antibiotique+booster))

Le booster est un terme générique pour désigner les composés potentialisateurs de l'invention, de formule (I).

Sauf indication contraire, les ratios indiqués dans les tableaux sont des ratios massiques.

***RATIOS MASSIQUES UTILISES***

- 64/500 correspond à 1 pour 8 environ (noté 1pour 8 dans les tableaux)
- 64/100 correspond à 1 pour 1,5 environ (noté 1pour 1,5 dans les tableaux)
- 64/50 correspond à 1 pour 0,75 environ (noté 1pour 0,75 dans les tableaux)
- 64/10 correspond à 1 pour 0,15 environ (noté 1pour 0,15 dans les tableaux)

**Description des protocoles de test / souches de test.**

- **souches bactériennes**

Les souches testées sont isolées de divers prélèvements humains (sang, urines, aspirations pulmonaires, etc..). Les souches étudiées dans les exemples suivants sont choisies parmi les souches du tableau suivant:

Tableau 2

| | |
|---|---|
| Enterobacteriaceae, n=11 | |
| Escherichia coli | Caractérisation phénotypique: BLSE, sauvage, pénicillinase, résistance aux fluoroquinolones, résistance à l'acide nalixidique. |
| Autres bacilles gram, n= 10 | |
| Pseudomonas aeruginosa | Caractérisation génotypique: BLSE, céphalosporinase, pénicillinase, absence de porine, multiresistance, sauvage |
| Staphylococcaceae, n= 10 | |
| Staphylococcus | Caractérisation phénotypique: résistance à la méthicilline, aux fluoroquinolone, à la kanamicine, à la tobramicine, multiresistance, sauvage |
| Streptococcus et app, n= 2 | |
| Enterococcus sp | Caractérisation phénotypique: résistance à l'érythromycine, à la clindamycine, à la pristinamicine, sauvage |

Les souches de levures testées (n=33) appartiennent aux espèces *Candidas albicans, Candida tropicalis, Candida krusei, Candida parapsilosis, Candida glabrata.*

Exemple 1 : Mesure des concentrations minimales inhibitrices

CMI DU CINEOLE SEUL (TOUS SOLVANTS)

Tableau 3

| | | Cinéole | | | |
|---|---|---|---|---|---|
| | | eau/ eau | Tween/eau | Tween/ prop glyc | DMSO |
| *Pyo* | *CMI 50* | >1 | >1 | >1 | 1 |
| | *CMI 90* | >1 | >1 | >1 | 1 |
| *Coli* | *CMI 50* | >1 | >1 | >1 | >1 |
| | *CMI 90* | >1 | >1 | >1 | >1 |
| *Staph* | *CMI 50* | >1 | >1 | >1 | >1 |
| | *CMI 90* | >1 | >1 | >1 | >1 |

Les produits sont testés en dilution successive de 1% à 0,00375 %. La valeur >1% indique qu'aucune inhibition bactérienne n'a été observée aux concentrations testées. Une valeur égale à 1,00% correspond à 10 000 mg/L (soit 10g/L).

***1/ Test aérobie vs anaérobie dans DMSO et Tween® 80***

On a testé l'effet de potentialisation de l'Augmentin® avec le cinéole sur différentes souches dans des conditions aérobie/anaérobie. Le ratio massique Augmentin®/cinéole est de 64 pour 100 (1 pour 1,5). On mesure les CMI sur différentes souches. Ensuite, on calcule sur les souches appartenant au même genre le gain en CMI.

Le gain en CMI exprime le ratio suivant : CMI (50 ou 90) avec potentialisateur / CMI (50 ou 90) sans potentialisateur. Les résultats sont reportés dans le tableau suivant :

Tableau 4

| | | aérobie | | anaérobie | |
|---|---|---|---|---|---|
| | | Tween® 80 | DMSO | Tween® 80 | DMSO |
| *E. Coli* | *Gain CMI 50* | 0,75 | 1,00 | 0,67 | 0,67 |
| | *Gain CMI 90* | 1,00 | 0,77 | 0,37 | 0,37 |
| *Stapyilloccoccus* | *Gain CMI 50* | 0,50 | 0,50 | 0,50 | 0,50 |
| | *Gain CMI 90* | 1,00 | 1,00 | 0,55 | 0,60 |

Ce test montre que le cinéole permet de potentialiser l'effet de l'Augmentin® sur de grandes séries de souches. L'effet de potentialisation s'exprime en conditions aérobie ou anaérobie et peut être influencé, par le solvant mis en jeu pour la dispersion des produits.

***2/ Test avec dilution directe dans l'eau, en aérobie***

Les même mesures et calculs sont réalisés dans l'eau, avec l'Augmentin® ou l'amoxicilline seule.

Les résultats sont reportés dans le tableau suivant :

Tableau 5

| | | Augmentin®/cinéole 1 : 1,5 | Augmentin®/cinéole 1 : 0,75 | amoxicilline/cinéole 1 : 1,5 |
|---|---|---|---|---|
| *E. Coli* | *Gain CMI 50* | 1,33 | 1,33 | 1,00 |

(suite)

| | | Augmentin®/cinéole 1 : 1,5 | Augmentin®/cinéole 1 : 0,75 | amoxicilline/cinéole 1 : 1,5 |
|---|---|---|---|---|
| *Staphyllococcus* | *Gain CMI 130* | 0,50 | 0,75 | 0,83 |
| | *Gain CMI 170* | 0,55 | 0,50 | 1,00 |

Ce test montre que le cinéole testé *in vitro* dans un autre solvant montre des effets de potentialisation légèrement différents. Ici la synergie n'est plus apparente sur E coli avec une dilution directe dans l'eau alors qu'elle est clairement visible dans un autre solvant (Ex1-1). Ainsi un résultat neutre dans un solvant ne présage pas d'un résultat neutre dans un autre solvant.

Ce test montre aussi que l'effet est ratio-dépendant.

***3 /Tests amoxicilline (AMX) et Augmentin® (AUG) dans Tween®80/eau vs DMSO***

Les résultats sont reportés dans le tableau suivant :

Tableau 6

| | | AMX/Cinéole 1 : 8 Tween eau | AMX/Cinéole 1 : 8 DMSO | AMX/ Cineole 1 : 1,5 Tween eau | AMX/ Cineole 1 : 1,5 DMSO | AMX/ Cineole 1 : 0,15 Tween eau | AMX/ Cineole 1 : 0,15 DMSO |
|---|---|---|---|---|---|---|---|
| *E. Coli* | *Gain CMI 50* | 2,00 | 1,33 | 2,00 | 1,33 | 2,00 | 2,00 |
| *Staphilloccoccus* | *Gain CMI 50* | 0,75 | 1,33 | 0,38 | 1,33 | 1,00 | 1,50 |
| | *Gain CMI 90* | 0,50 | 1,00 | 1,00 | 1,00 | 0,50 | 1,00 |

Ici encore des potentialisations semblent disparaître selon le type bactérien et selon le solvant. Le tween associé à l'eau permet une mise en valeur claire de la potentialisation de l'amoxicilline par le cinéole.

Les résultats sont reportés dans le tableau suivant :

Tableau 7

| | | Aug/ Cineole 1: 8 | | Aug/ Cineole 1: 1,5 | | Aug/ Cineole 1: 0,15 | |
|---|---|---|---|---|---|---|---|
| | | Tween eau | DMSO | Tween eau | DMSO | Tween eau | DMSO |
| *Coli* | *Gain CMI 50* | 1,00 | 2,00 | 0,75 | 2,00 | 1,00 | 2,00 |
| | *Gain CMI 90* | 0,77 | 2,00 | 1,00 | 1,00 | 0,77 | 1,30 |
| *Staph.* | *Gain CMI 50* | 1,00 | 1,00 | 0,50 | 0,50 | 1,00 | 1,00 |
| | *Gain CMI 90* | 1,00 | 1,00 | 1,00 | 1,00 | 1,00 | 2,00 |

La dilution de l'Augmentin® dans l'eau et le tween permet la mise en évidence de l'effet de potentialisation à des doses faibles (jusque 0,025 mg/l de cinéole) puisque la CMI de l'Augmentin® seul sur ces souches peut être de 0,25 mg/l sur certains staphylocoques..

***4/Tests CIP (ciprofloxacin) dans Tween®80/eau, vs DMSO***

Les résultats sont reportés dans le tableau suivant :

Tableau 8

| | | CIP / Cinéole 1 :8 | | CIP / Cinéole 1 :1,5 | | CIP / Cinéole 1:0,015 | |
|---|---|---|---|---|---|---|---|
| | | Tween eau | DMSO | Tween eau | DMSO | Tween eau | DMSO |
| *Pyo.* | *Gain CMI 90* | 0,91 | 0,55 | 1,82 | 0,50 | 1,00 | 0,50 |
| *Coli* | *Gain CMI 90* | 0,53 | 0,30 | 0,53 | 0,27 | 0,60 | 0,27 |
| *Staph.* | *Gain CMI 90* | 1,00 | 0,19 | 1,16 | 1,43 | 1,16 | 1,43 |

Le test montre que le cinéole potentialise aussi la ciprofloxacine à des doses faibles de cinéole (des synergies sont observées à des doses de 0,125 mg de cinéole). Le solvant et le ratio ont tous les deux une influence sur le résultat.

***5/Tests Tetratcycline (TE) dans eau, tween, TE, T/P***

Les résultats sont reportés dans le tableau suivant :

Tableau 9

| | | TE / Cineole 1 :1,5 Tween Eau | TE / Cineole 1 :1,5 Tween Propylène Glycol | TE / Cineole 1 :1,5 DMSO | TE / Cineole 1 : 1,5 Eau |
|---|---|---|---|---|---|
| *Pyo* | *Gain CMI 50* | 0,50 | 2,00 | 0,67 | 1,50 |
| | *Gain CMI 90* | 1,00 | 1,00 | 0,50 | 1,00 |
| *Coli* | *Gain CMI 50* | 1,00 | 2,00 | 1,00 | 1,00 |
| | *Gain CMI 90* | 0,50 | 1,20 | 1,00 | 1,00 |
| *Staph* | *Gain CMI 50* | 1,00 | 1,00 | 1,00 | 1,00 |
| | *Gain CMI 90* | 0,91 | 1,00 | 0,91 | 1,00 |

Le test démontre la capacité du cinéole à potentialiser la tétracycline, notamment dans le DMSO et le mélange Tween-eau.

***6/Tests GEN (gentamycine) dans EAU/ Tween®80+eau / Tween®80 + Propylèneglycol***

Les résultats sont reportés dans le tableau suivant :

Tableau 10

| | | eau | | Tween | | Tween dilution prop glycol | |
|---|---|---|---|---|---|---|---|
| | | GEN / Cineole 1 :8 | GEN / Cineole 1 :1,5 | GEN / Cineole 1 :8 | GEN / Cineole 1 :1,5 | GEN / Cineole 1 :8 | GEN / Cineole 1 : 1,5 |
| *Pyo* | *Gain CMI 50* | 1,00 | 1,00 | 1,00 | 1,00 | 2,00 | 2,00 |
| | *Gain CMI 90* | 1,00 | 1,11 | 1,00 | 0,90 | 3,98 | 1,00 |
| *Coli* | *Gain CMI 50* | 1,00 | 2,00 | 1,00 | 1,00 | 2,00 | 0,50 |
| | *Gain CMI 90* | 1,00 | 1,00 | 1,00 | 0,55 | 2,00 | 0,50 |
| *Staph* | *Gain CMI 50* | 1,00 | 2,00 | 1,00 | 1,00 | 1,00 | 0,50 |
| | *Gain CMI 90* | 1,00 | 1,25 | 1,50 | 1,50 | 2,00 | 0,83 |

Le test démontre la capacité du cinéole à potentialiser la gentamycine, notamment dans le mélange tween + propylène.

***7/ Sertaconazole dans le DMSO***

L'effet de potentialisation du cinéole sur des levures candida albicans est également testé. Les résultats sont reportés dans le tableau suivant :

Tableau 11

| | Sertaconazole + cinéole 1 : 1,5 - DMSO |
|---|---|
| *Gain CMI 90* | 0,5 |
| *Gain CMI 50* | 0,25 |

Ce test démontre que le cinéole est en mesure de potentialiser le sertaconazole.

**Exemple 2** : **test de double dilution**

Le test de double dilution est un test de mesure de CMI réalisé sur des dilutions successibles des 2 produits de la composition. Ainsi 12 dilutions du booster (cinéole) sont testées avec 12 dilutions de l'antibiotique (Amoxicilline). Le tableau récapitulatif exprime pour chaque souche et pour chaque dilution du booster la CMI mesurée de l'Antibiotique.

| | | en CMI de cineole (en mg/L) | | | | | | | | | | | | | | |
|---|---|---|---|---|---|---|---|---|---|---|---|---|---|---|---|---|
| | | 50000 | 25000 | 10000 | 5000 | 2500 | 1250 | 600 | 300 | 150 | 75 | 37,5 | 20 | 10 | Amox | |
| Nom | Référence | 5 | 2,5 | 1 | 0,5 | 0,25 | 0,125 | 0,06 | 0,03 | 0,015 | 0,0075 | 0,00375 | 0,002 | 0,001 | 0 | Cineole seul |
| E.coli | ATCC 25922 | 0,06 | 4 | * | 8 | 16 | 8 | 8 | 8 | 8 | 8 | 8 | 8 | 0,5 | 8 | >2,5 |
| | 8137 | 0,25 | 4 | * | 8 | 8 | 8 | 8 | 8 | 8 | 8 | 8 | 8 | 0,5 | 8 | >2,5 |
| | 8151 | 0,25 | 4 | 4 | 8 | 8 | 8 | 8 | 8 | 8 | 8 | 8 | 8 | 0,5 | 8 | >2,5 |
| | 8155 | 0,25 | 4 | 8 | 8 | 8 | 8 | 8 | 8 | 8 | 8 | 8 | 8 | 0,5 | 8 | >2,5 |
| | 8156 | 4 | 8 | 8 | 8 | 16 | 8 | 8 | 8 | 8 | 8 | 8 | 8 | 0,5 | 8 | >2,5 |
| Staphylococcus | 8148 | 0,25 | 16 | 4 | 8 | 8 | 8 | 8 | 8 | 8 | 8 | 8 | 8 | 0,5 | 8 | >2,5 |

Tableau 12

Le code couleur permettant d'interpréter les résultats est le suivant : aucune couleur si la CMI est équivalent avec et sans booster, gris si la valeur est inférieure avec booster. On constate que le cinéole est capable de potentialiser (phénomènes additifs) l'amoxycilline à des concentrations très élevées (CMI 50 000mg/L). Lorsque la concentration en cinéole diminue, il n'y a plus de phénomènes potentialisation. D'une manière surprenante, cette potentialisation réapparait lorsque la dose en cinéole est très faible.

Des essais ont également été réalisés avec l'augmentin® (amoxycilline/acide clavulanique) où on observe les mêmes résultats. Un isobologramme établissant la courbe de la CMI d'amoxicilline observée en fonction de la concentration (dilution) de cinéole (souche : SA8238) est reporté sur la figure 1. On constate une synergie à des doses de cinéole importantes et faibles, avec un *discontinuum* inattendu.

**Exemple 3** : **etest**

Les tests correspondant sont effectués sur la base du test de CMI usuel : la bandelette etest® (Biomérieux) est déposée à la surface d'une gélose ensemencée dans laquelle le booster a été incorporé. Ce test permet de minimiser les risques d'effet solvant, à savoir une éventuelle interaction avec le solvant utilisé *in vitro.*
On mesure la CMI de l'antibiotique seul (témoin Tween eau ou témoin DMSO), ou en association avec le cinéole à trois concentrations différentes (1mg/L, 4mg/L, 16mg/L) dans différents solvants (Tween®80 ou DMSO).
Le témoin correspond au test réalisé sans cineole. Un témoin est fait dans chaque solvant testé : Eau distillée, Tween/eau et DMSO.

Témoin ED = Antibiotique seul dans Eau distillée

Témoin DMSO = antibiotique seul dans DMSO

Témoin TE = antibiotique seul dans Tween® 80

Sur ces souches, utilisé seul, le cinéole a une CMI50 supérieure à 10 000 mg/L.
Dans les tableaux, le code couleur permettant d'interpréter les résultats est le suivant : aucune couleur si la CMI est équivalent avec et sans booster, gris si la valeur est inférieure avec booster.

| | Coli 8150 | | | Coli 8138 (pénicillinase) | | MRSA 10178 | | | |
|---|---|---|---|---|---|---|---|---|---|
| CMI en mg/L | Tigecycline | Pipera tazo | Meropenem | Cefoxitine | Pipera tazo | Cefoxitine | Tigecycline | Clindamycine | Penicilline G |
| temoin ED | 0,125 | 0,75 | 0,047 | 3 | 6 | 6 | 0,125 | 0,094 | 1 |
| temoin Tween eau | 0,125 | 1 | 0,094 | 6 | 6 | 6 | 0,125 | 0,19 | 0,5 |
| Témoin DMSO | 0,19 | 1,5 | 0,094 | 8 | 16 | 6 | 0,25 | 0,064 | 1,5 |
| Cineole 1mg Tween/eau | 0,125 | 1,5 | 0,064 | 3 | 3 | 4 | 0,125 | 0,064 | 0,5 |
| Cineole 1mg DMSO | 0,19 | 1 | 0,064 | 8 | 6 | 4 | 0,19 | 0,094 | 0,75 |
| Cineole 4mg Tween/eau | 0,094 | 0,75 | 0,047 | 3 | 6 | 3 | 0,094 | 0,064 | 0,38 |
| Cineole 4mg DMSO | 0,125 | 1 | 0,064 | 3 | 8 | 4 | 0,19 | 0,064 | 0,5 |
| Cineole 16mg Tween/eau | 0,1 | 0,1 | 0,1 | 1 | 2 | 1 | 0,094 | 0,047 | 0,125 |
| Cineole 16mg DMSO | 0,064 | 0,75 | 0,064 | 3 | 6 | 4 | 0,125 | 0,047 | 0,19 |

Tableau 13

D'autres e-tests sont également réalisés en DMSO avec l'amoxicilline en présence ou non de cinéole (absence de cinéole = témoin). Les tests sont reportés dans le tableau suivant :

| | | Témoin ED | Cinéole 16 mg/L | Cinéole 4 mg/L |
|---|---|---|---|---|
| | | Amox | Amox | Amox |
| | ATCC25922 | 16 | 12 | 12 |
| *E.coli* | 8141 | 8 | 0,1 | 6 |
| | 8150 | 8 | 0,1 | 6 |
| | 8156 | 12 | 8 | 8 |
| *Staphylococcus* | 8143 | 2 | 1,5 | 1,5 |
| | 8146 | 0,5 | 0,19 | 0,38 |
| | 8149 | 1 | 0,38 | 0,5 |
| | 8240 | 24 | 16 | 12 |
| | 8241 | 32 | 12 | 8 |
| | 10168 | 2 | 1 | 1 |

Tableau 14

Une potentialisation est observée sur toutes les souches.

D'autres e-tests sont également réalisés avec l'amoxycilline/acide clavulanique (Augmentin®) en présence ou non de cinéole (témoin: pas de cinéole, CIN16 : cinéole à 16mg/L, CIN4 : cinéole à 4mg/L) DMSO. Les tests sont reportés dans le tableau suivant :

| | | Témoin ED | CIN16 | CIN4 |
|---|---|---|---|---|
| | ATCC25922 | 12 | 6 | 8 |
| *E.coli* | 8141 | 6 | 0,1 | 4 |
| *Staphylococcus* | 8146 | 0,38 | 0,19 | 0,25 |
| | 8149 | 0,75 | 0,38 | 0,5 |
| | 8238 | 1 | 0,75 | 0,38 |
| | 8239 | 6 | 6 | 3 |
| | 8240 | 4 | 3 | 3 |
| | 8241 | 12 | 12 | 8 |
| | 10168 | 0,75 | 0,5 | 0,38 |

Tableau 15

D'autres e-tests sont également réalisés sur des Pasteurelles. Les résultats sont rapportés dans les tableaux suivants :

Tableau 16

| | 14012 | | | | |
|---|---|---|---|---|---|
| | Amox | Augmentin | ciprofloxacine | meropenem | cefoxitine |
| Témoin ED | 0,032 | 0,094 | 0,004 | 0,008 | 0,5 |
| Témoin DMSO | 0,094 | 0,094 | 0,006 | 0,008 | 0,5 |
| Témoin TE | 0,19 | 0,19 | 0,006 | 0,023 | 0,75 |
| Cineole 5mg eau | 0,094 | 0,094 | 0,003 | 0,006 | 0,5 |
| Cineole 5mg DMSO | 0,094 | 0,094 | 0,004 | 0,006 | 0,5 |
| Cineole 5mg TE | 0,094 | 0,094 | 0,004 | 0,008 | 0,5 |
| Cineole 10 mg eau | 0,094 | 0,125 | 0,004 | 0,008 | 0,5 |
| Cineole 10 mg DMSO | 0,125 | 0,125 | 0,004 | 0,006 | 0,38 |
| Cineole 10 mg TE | 0,094 | 0,125 | 0,006 | 0,008 | 0,5 |

Tableau 17

| | 14013 | | |
|---|---|---|---|
| | Amox | Augmentin | ciprofloxacine |
| Témoin ED | 0,19 | 0,19 | 0,012 |
| Témoin DMSO | 0,19 | 0,25 | 0,012 |
| Témoin TE | 0,25 | 0,25 | 0,016 |
| Cineole 5mg eau | 0,25 | 0,25 | 0,012 |
| Cineole 5mg DMSO | 0,25 | 0,19 | 0,012 |
| Cineole 5mg TE | 0,25 | 0,25 | 0,012 |
| Cineole 10 mg eau | 0,19 | 0,19 | 0,016 |
| Cineole 10 mg DMSO | 0,25 | 0,19 | 0,012 |
| Cineole 10 mg TE | 0,125 | 0,19 | 0,012 |

Tableau 18

| | 14014 | | |
|---|---|---|---|
| Témoin ED | Amox | meropenem | cefoxitine |
| Témoin DMSO | 0,125 | 0,016 | 0,38 |
| Témoin TE | 0,094 | 0,012 | 0,5 |
| Cineole 5mg eau | 0,19 | 0,016 | 0,5 |
| Cineole 5mg DMSO | 0,094 | 0,012 | 0,19 |
| Cineole 5mg TE | 0,094 | 0,016 | 0,38 |
| Cineole 10 mg eau | 0,125 | 0,016 | 0,38 |
| Cineole 10 mg DMSO | 0,125 | 0,016 | 0,5 |
| Cineole 10 mg TE | 0,094 | 0,012 | 0,38 |
| Témoin ED | 0,094 | 0,012 | 0,38 |

**Exemple 4 : test de croissance / test de bactéricidie**

***1 Test de croissance sur la souche 10168***

Les tests de croissance sont faits en milieu liquide avec une dispersion du booster des antibiotiques préalable dans un solvant adapté.

On mesure la cinétique de croissance des bactéries en présence d'Augmentin®, d'Augmentin® boosté cinéole ou de cinéole.

Les résultats sont reportés sur la figure 2 (souche 10168, genre Staphylococcus), dont la légende est :

Carré : témoin (bactérie seule)
Triangle : Augmentin® dilué dans un mélange Tween®80/eau à une concentration de 4 fois sa CMI
Croix (×) : Augmentin® et le cinéole, dilués dans un mélange Tween®80/eau, l'Augmentin® est à une concentration de 4 fois sa CMI, ratio massique Augmentin® / cinéole de 64/100
Etoile (*) : cinéole seul dilué dans un mélange Tween®80/eau.

On constate que la présence de cinéole, à une très faible dose, permet d'accélérer la vitesse de bactéricidie de l'Augmentin®.

***2 Effet post antibiotique sur la souche 08150 (CMI de l'Augmentin®= 4 mg/L)***

On compte le nombre de bactéries après 16h de contact en présence d'augmentin® (AC) et/ou de cinéole, puis plusieurs heures après la fin de mise en contact. L'Augmentin® est utilisé à sa CMI. Les résultats sont reportés dans le tableau suivant :

Tableau 19

| | temps de contact de l'augmentin®+cineole de 16h | | | | | |
|---|---|---|---|---|---|---|
| temps (h) | témoin | AC CMI | AC CMI Cinéole 16mg/L | AC CMI Cinéole 4mg/L | Cinéole 16mg/L | Cinéole 4mg/L |
| 0 | 2,47E+07 | 2,53E+05 | 1,40E+04 | 1,00E+02 | 2,20E+07 | 2,17E+07 |
| 1 | 9,20E+06 | 3,40E+04 | 7,80E+03 | 9,00E+03 | 3,88E+07 | 4,92E+07 |
| 2 | 7,10E+07 | 1,60E+05 | 5,10E+04 | 3,00E+03 | 6,40E+07 | 5,50E+07 |
| 3 | 2,07E+08 | 1,84E+06 | 7,60E+05 | 3,60E+04 | 2,70E+08 | 3,90E+08 |
| 4 | 3,10E+08 | 9,90E+06 | 6,40E+06 | 3,40E+05 | 4,20E+08 | 3,50E+08 |
| 5 | 6,60E+08 | 3,70E+07 | 3,55E+07 | 2,37E+06 | 5,50E+08 | 5,90E+08 |
| 6 | 4,10E+08 | 2,10E+08 | 2,00E+08 | 1,27E+07 | 5,90E+08 | 6,00E+08 |
| Témoin = Absence de cineole et d'Augmentin®. | | | | | | |

On constate que l'ajout de cinéole permet de réduire significativement le nombre de bactéries, y compris après la mise en contact.

***3 Effet post antibiotique sur la souche 08152 (CMI de l'Augmentin®= 0,5mg/L)***

On compte le nombre de bactéries après 16h de contact en présence d'augmentin® (AC) et/ou de cinéole, puis plusieurs heures après la fin de mise en contact. L'Augmentin® est utilisé à une concentration de 4 fois sa CMI. 4XCMI signifie que la concentration en antibiotique est 4 fois supérieure à sa CMI, mesurée lorsqu'il est utilisé seul sur la souche considérée. Les résultats sont reportés dans le tableau suivant :

Tableau 20

| Temps (h) | témoin | AC 4x CMI | AC 4xCMI Cinéole 16mg/L | AC4xCMI Cinéole 4mg/L | Cineole 16mg/L | Cineole 4mg/L |
|---|---|---|---|---|---|---|
| 0 | 1,24E+07 | 5,50E+02 | 2,90E+02 | 1,50E+02 | 9,00E+06 | 8,00E+06 |

(suite)

| Temps (h) | témoin | AC 4x CMI | AC 4xCMI Cinéole 16mg/L | AC4xCMI Cinéole 4mg/L | Cineole 16mg/L | Cineole 4mg/L |
|---|---|---|---|---|---|---|
| 1 | 9,30E+06 | 1,18E+03 | 5,50E+02 | 3,00E+02 | 1,64E+07 | 1,50E+07 |
| 2 | 2,74E+07 | 1,19E+03 | 4,60E+02 | <10E01 | 5,50E+06 | 1,78E+07 |
| 3 | 3,60E+07 | 3,50E+03 | 3,40E+02 | 3,90E+02 | 7,50E+07 | 5,60E+07 |
| 4 | 2,16E+07 | 8,10E+03 | 1,59E+03 | 1,80E+03 | 5,90E+08 | 7,30E+08 |
| 5 | 2,00E+08 | 9,60E+04 | 2,30E+03 | 3,10E+03 | 3,42E+08 | 3,44E+08 |
| 6 | 2,50E+08 | 3,40E+04 | 1,32E+04 | 1,40E+04 | 5,70E+08 | 5,30E+08 |
| 24 | 3,70E+08 | 4,50E+08 | 4,60E+08 | 2,30E+08 | 9,70E+08 | 9,30E+08 |
| Témoin = absence de cinéole et d'Augmentin® | | | | | | |

On constate que l'ajout de cinéole permet de réduire significativement le nombre de bactéries, y compris après la mise en contact.

**Exemple 5** : **résistance**

Cent µl d'une culture en bouillon Mueller Hinton de la souche à étudier (inoculum lourd >$10^{10}$ UFC/ml) sont étalés sur une boîte de Mueller Hinton contenant une concentration d'huile égale à 4 fois la CMI du produit à tester. Après 48h d'incubation on observe la présence ou non de colonies susceptibles d'être des mutants résistants. On a constaté que l'utilisation de cinéole permettait également de diminuer l'apparition de bactéries résistantes.

Les résultats sont reportés dans les tableaux suivants :

Tableau 21 - Souche 10168 / MRSA

| Témoin ED | Témoin DMSO |
|---|---|
| envahie | envahie |
| Ciprofloxacine DMSO | Ciprofloxacine / cinéole 1:1,5 DMSO |
| envahie | 0 |

Alors que la ciprofloxacine seule sur le *S. aureus* résistant à la méthicilline (MRSA) entraine l'apparition d'innombrable mutants, l'ajout de cinéole à la ciprofloxacine n'entraine plus l'apparition des mutants

Tableau 22 - Souche 9003 / BLSE

| Témoin ED | Témoin DMSO |
|---|---|
| envahie | envahie |
| Ciprofloxacine DMSO | Ciprofloxacine / cinéole 1 :1,5 DMSO |
| Nombreux | 26 |

Là encore la cinéole, entraine la diminution d'apparition de mutants sur une souche à gram négatif résistante.

**Exemple 6** : **Modèle de sepsis**

200 µL d'une solution bactérienne (MRSA) ajustée à 5.108 CFU/ml est injectée par voie intraveineuse (voie retro-orbitale) à des souris CD1. Le traitement est commencé 1 h après l'injection bactérienne par voie sous cutanée.

A 24 h, les souris sont euthanasiées et un comptage bactérien est effectué dans le rein. Test Vivo 1 : injection d'amoxicilline :acide clavulanique (AMC) à dose variable (0,25 mg/kg, 2 mg/kg, 16 mg/kg et 128 mg/kg)+ cinéole (B) à dose constante (30 mg/kg), injectés 2 fois par 24 (H+1 et H+4). Témoins : groupe non traité et groupe recevant 30 mg/kg de cinéole sans antibiotique. Nombre de bactéries log10 CFU/g rein

Les résultats sont reportés sur la figure 3.

Test vivo 2 Injection d'amoxicilline : clavulanique (AMC) à dose constante (8mg/kg) + cineole (B) à dose variable (4 mg/kg, 64 mg/kg, 128 mg/kg, 256 mg/kg, 512 mg/kg) injectés 2 fois par jour. Témoins : groupe non traité, groupe recevant 32 mg/kg de cinéole sans antibiotique (CIN), groupe recevant 8 mg/kg d'antibiotique sans cinéole (AMC8).

(*) p<0,05 vs control, AMC8 et CIN
(£)p<0,05 vs control et CIN
($)p<0,05 vs control, AMC8, CIN, AMC+CIN 128, AMC+CIN 256

Les résultats sont reportés sur la figure 4 et également dans le tableau suivant :

Tableau 23

| Régime thérapeutique | Mean ± SD log10 CFU/g de rein |
|---|---|
| Contrôle | 6,27±0,27 |
| AMC, 8mg/kg | 5,92±0,36 |
| CIN, 32 mg/kg | 6,35±0,29 |
| AMC + CIN, 4 mg/kg | 5,42±0,32 * |
| AMC + CIN, 64 mg/kg | 5,61±0,44 £ |
| AMC + CIN, 128 mg/kg | 5,90±0,32 |
| AMC + CIN, 256 mg/kg | 6,06±0,64 |
| AMC + CIN, 512 mg/kg | 4,87±0,47 $ |

On constate de nouveau une potentialisation à faible dose ou à forte dose, sans potentialisation dans l'intervalle. Ce qui est en ligne avec les résultats *in vitro* qui démontrent de façon surprenante que la synergie avec le cinéole apparait à dose faible (et compatible avce l'usage médicale) et à dose très élevées (incompatible avec l'usage médical). Entre ces extrêmes de dose, et comme cela avait été le cas *in vitro,* apparaît de façon surprenante une plage de concentrations dans laquelle l'effet de potentialisation est absent.

**Exemple 7 : Efficacité antibactérienne du plasma**

On injecte (sc.) à des souris Balb/c, le produit antimicrobien ou le mélange antimicrobien + booster. Les doses administrées sont les suivantes:

Groupe 1: Gentamycine (GE) 6 mg/kg
Groupe 2: Gentamycine 6 mg/kg + cinéole 30 mg/kg
Groupe 3: amoxicilline (AMX) 30 mg/kg
Groupe 4: Amoxicilline 30 mg/ kg + 30 mg/kg cinéole
Groupe 5 Augmentin® (AMC) 30 mg/kg
Groupe 6 Augmentin® 30 mg/kg + 30 mg/kg cinéole.

A différents temps post-injection, les souris sont euthanasiées, et le sang prélevé, hépariné et centrifugé pour en extraire le plasma.

Une série de dilutions successives (1/3) du plasma est réalisée et une goutte de ces différentes concentrations de plasma est déposée sur un milieu Baird Parker ensemencé (MSSA 8238 pour les groupes GE et AMX, MRSA 10168 pour le groupe AMC). Les effets antibactériens sont mesurés après 24 d'incubation. Les dilutions successives permettent de déterminer l'effet antimicrobien et le rapporter à la CMI. Le ratio concentration efficace / CMI est ainsi déterminé

Les résultats sont reportés dans les tableaux suivants :

Tableau 24

| c/MIC | 0,083h | 1h | 2h | 5h | 8h | 24h | **AUC 0-24h** | Temps passé au dessus de la CMI |
|---|---|---|---|---|---|---|---|---|
| Moyenne groupe 1 | 10 | 3 | 4 | 0,2 | 0,2 | 0,155 | 19,2005 | < 5h |

(suite)

| c/MIC | 0,083h | 1h | 2h | 5h | 8h | 24h | **AUC 0-24h** | Temps passé au dessus de la CMI |
|---|---|---|---|---|---|---|---|---|
| Moyenne groupe 2 | 2 | 5,4 | 1,5 | 12,65 | 1,15 | 2 | 73,9679 | > 24h |
| Moyenne groupe 3 | 25 | 3,5 | 2,45 | 0,1505 | 0,5005 | 0,3 | 27,3235 | < 5h |
| Moyenne groupe 4 | 30 | 7 | 1,95 | 1,2 | 0,95 | 1,1 | 45,7895 | > 24h |

Tableau 25

| | 0,25 h | 0,5 h | 1 h | 2 h | 4 h | 8 h | 24 h | AUC 0-24h | Temps passé au dessus de la CMI |
|---|---|---|---|---|---|---|---|---|---|
| Moyenne groupe 5 | 5,5 | 1,75 | 2,15 | 1,75 | 0,3 | 0,3 | 0,01 | 9,56125 | <4h |
| Moyenne groupe 6 | 6,5 | 2 | 1,65 | 3,15 | 2 | 1,15 | 1,1 | 33,825 | >24h |

Le rapport de la concentration plasmatique rapporté à la CMI est obtenu par les dilutions successives du plasma. Bien que cette méthode soit semi quantitative, elle permet néanmoins de montrer que l'effet est plus intense quand le cinéole est présent (AUC supérieure). De même et de façon univoque, l'ajout de cinéole permet d'allonger le temps passé au dessus de la CMI.

Ce résultat est en ligne avec la courbe de la figure 2, réalisée *in vitro.*

Liste des abréviations :

| | |
|---|---|
| Amox | amoxycilline |
| Aug | augmentin® |
| Staph | Staphylococcus |
| Coli | E. coli |
| Pyo | Pseudomonas aeruginosa |
| AUC | aire sous la courbe |

## Revendications

**1.** Association d'un agent antimicrobien et d'un composé de formule I pour son utilisation dans le traitement d'une infection microbienne, le composé répond à la formule I suivante :

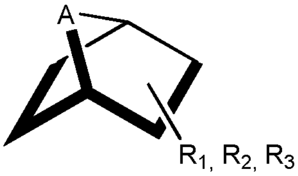

(I)

dans laquelle $R_1$, $R_2$, $R_3$ représentent chacun, indépendamment l'un de l'autre, H, OH un groupe ($C_1$-$C_6$,)alkyle, ($C_2$-$C_6$)alcényle, ($C_1$-$C_6$)halogénoalkyle, ($C_1$-$C_6$,)alcoxy, ($C_2$-$C_6$)alcénoxy, ($C_1$-$C_6$)halogénoalcoxy, -O-CO-($C_1$-$C_6$)alkyle ;
A représente un hétéroatome ou un groupe -(Het)-R' ou R-(Het)-R' où R, R', représentent chacun, indépendamment l'un de l'autre, un groupe ($C_1$-$C_4$)alcanediyle éventuellement substitué par des radicaux alkyles en

$C_1$-$C_4$ et Het représente un hétéroatome.
en un ratio massique, composé de formule (I) : agent antimicrobien, variant de 8 :1 à 1 :10, en ce que l'agent antimicrobien n'est pas un terpénoïde ou un phenylpropanoide
et en ce que l'infection microbienne est induite notamment par un pathogène de souche A et que sur cette souche A, la concentration en composé (I) répond à l'équation suivante :

$$[C] < [CMI] / x$$

Où [C] est la concentration selon l'invention en composé (I) à utiliser sur la souche A
[CMI] est la CMI mesurée pour le composé (I), seul, sur cette souche A x est supérieur ou égal à 100, avantageusement à 1 000, plus avantageusement x est compris entre 2 000 et 10 000, voire supérieur à 50 000,

en ce que ledit composé de formule (I) est adapté pour une administration par voie systémique.

2. Association pour son utilisation selon la revendication 1, **caractérisée en ce que** dans la formule (I) $R_2$ et $R_3$ représentent chacun H, $R_1$ représente un groupe ($C_1$-$C_6$)alkyle, et A représente l'hétéroatome O un groupe R-O-R' ou -O-R' où R, R', représentent chacun, indépendamment l'un de l'autre, un groupe ($C_1$-$C_2$)alcanediyle éventuellement substitué par des radicaux alkyles en $C_1$-$C_4$.

3. Association pour son utilisation selon la revendication 1 ou 2, **caractérisée en ce que** le composé de formule (I) est le cinéole.

4. Association pour son utilisation selon l'une des revendications précédentes, **caractérisée en ce que** ledit antimicrobien est un antibiotique, en particulier un antibiotique choisi parmi : les antibiotiques inhibant la synthèse du peptidoglycane, les antibiotiques inhibant la synthèse des acides nucléiques, les antibiotiques inhibant la synthèse des folates, les antibiotiques inhibant la synthèse des acides mycoliques, l'un quelconque de leurs sel pharmaceutiquement acceptables, et l'une quelconque de leurs combinaisons.

5. Association pour son utilisation selon l'une quelconque des revendications précédentes, **caractérisée en ce que** l'antimicrobien est un antifongique, en particulier un antifongique choisi parmi : les polyènes, les azolés, les allylamines, les thiocarbamates, les echinocandines, la griseofulvine, et la fluorocytosine.

6. Association pour son utilisation selon l'une des revendications précédentes dans laquelle le ratio massique composé de formule (I) : agent antimicrobien varie de 4 :1 à 1 :10, avantageusement de 1 :1 à 1 :10, plus avantageusement de 1 :1 à 1 :5.

7. Association pour son utilisation selon l'une des revendications précédentes, **caractérisée en ce que** l'infection microbienne est induite par un pathogène choisi parmi les genres potentiellement pathogènes suivants: *Acetobacter, Acetobacterium, Acinetobacter, Citrobacter, Enterobacter, Enterococcus, Escherichia, Helicobacter, Klebsiella, Proteus, Providencia, Pseudomonas, Salmonella, Serratia*, *Staphylococcus, Streptococcus, Actinobacillus, Neisseria, Mannheima, Pasteurella, Candida, Aspergillus, Cryptococcus Trichosporon, Malassezia,* et *Mycobacterium.*

8. Association pour son utilisation selon l'une des revendications précédentes, **caractérisée en ce que** l'antimicrobien est l'amoxicilline.

9. Association pour son utilisation selon l'une des revendications précédentes, **caractérisée** en ce l'antimicrobien est un mélange amoxicilline/ acide clavulanique.

10. Association pour son utilisation selon l'une des revendications précédentes, **caractérisée** en ce l'antimicrobien est le sertaconazole.

## Patentansprüche

1. Assoziation eines antimikrobiellen Mittels und einer Verbindung der Formel I zu deren Verwendung in der Behandlung einer mikrobiellen Infektion, wobei die Verbindung der folgenden Formel I entspricht:

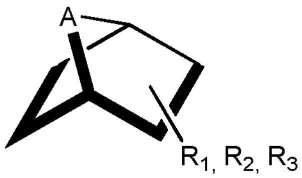

(I)

wobei $R_1$, $R_2$, $R_3$ jeweils unabhängig voneinander für H, OH, eine ($C_1$-$C_6$)-Alkyl-, ($C_2$-$C_6$)-Alkenyl-, ($C_1$-$C_6$)-Halogenalkyl-, ($C_1$-$C_6$)-Alkoxy-, ($C_2$-$C_6$)-Alkenoxy-, ($C_1$-$C_6$)-Halogenoalkoxy-, -O-CO-($C_1$-$C_6$)-Alkyl-Gruppe stehen;
A für ein Heteroatom oder eine -(Het)-R'- oder R-(Het)-R'-Gruppe steht, wobei R, R' jeweils unabhängig voneinander für eine ($C_1$-$C_4$)-Alkandiyl-Gruppe stehen, die gegebenenfalls durch $C_1$-$C_4$-Alkylreste substituiert ist, und Het für ein Heteroatom steht. in einem Massenverhältnis Verbindung der Formel (I) : antimikrobielles Mittel, das von 8 : 1 bis 1 : 10 variiert, dadurch, dass das antimikrobielle Mittel kein Terpenoid oder Phenylpropanoid ist, und dadurch, dass die mikrobielle Infektion vor allem durch einen Erreger des Stamms A hervorgerufen wird und dass auf diesen Stamm A die Konzentration an Verbindung (I) der folgenden Gleichung entspricht:

$$[K] < [MHK] / x$$

wobei [K] die erfindungsgemäße Konzentration an Verbindung (I) ist, die auf den Stamm A zu verwenden ist, [MHK] die gemessene MHK nur für die Verbindung (I) auf diesen Stamm A ist, x größer oder gleich 100, vorteilhafterweise als 1 000 ist, noch vorteilhafter x zwischen 2 000 und 10 000, ja sogar 50 000 liegt,

dadurch, dass die Verbindung der Formel (I) für eine systemische Verabreichung geeignet ist.

2. Assoziation zu deren Verwendung nach Anspruch 1, **dadurch gekennzeichnet, dass** in der Formel (I) $R_2$ und $R_3$ jeweils für H stehen, $R_1$ für eine ($C_1$-$C_6$)-Alkylgruppe steht, und A für das Heteroatom O, eine R-O-R'- oder -O-R'-Gruppe steht, wobei R, R' jeweils unabhängig voneinander für eine ($C_1$-$C_2$)-Alkandiyl-Gruppe stehen, die gegebenenfalls durch $C_1$-$C_4$-Alkylreste substituiert ist.

3. Assoziation zu deren Verwendung nach Anspruch 1 oder 2, **dadurch gekennzeichnet, dass** die Verbindung der Formel (I) Cineol ist.

4. Assoziation zu deren Verwendung nach einem der vorstehenden Ansprüche, **dadurch gekennzeichnet, dass** das antimikrobielle Mittel ein Antibiotikum ist, insbesondere ein Antibiotikum, ausgewählt aus: den Antibiotika, die die Peptidoglycan-Synthese hemmen, den Antibiotika, die die Nukleinsäure-Synthese hemmen, den Antibiotika, die die Folat-Synthese hemmen, den Antibiotika, die die Mykolsäure-Synthese hemmen, einem deren pharmazeutisch verträglichen Salze und einer deren Kombinationen.

5. Assoziation zu deren Verwendung nach einem der vorstehenden Ansprüche, **dadurch gekennzeichnet, dass** das antimikrobielle Mittel ein Antimykotikum ist, insbesondere ein Antimykotikum, ausgewählt aus: Polyenen, Azolen, Allylaminen, Thiocarbamaten, Echinocandinen, Griseofulvin und Fluorcytosin.

6. Assoziation zu deren Verwendung nach einem der vorstehenden Ansprüche, wobei das Massenverhältnis Verbindung der Formel (I) : antimikrobielles Mittel von 4 : 1 bis 1 : 10, vorteilhafterweise von 1 : 1 bis 1 : 10, noch vorteilhafter von 1 : 1 bis 1 : 5 variiert.

7. Assoziation zu deren Verwendung nach einem der vorstehenden Ansprüche, **dadurch gekennzeichnet, dass** die mikrobielle Infektion von einem Erreger hervorgerufen wird, ausgewählt aus den folgenden potentiell krankheitserregenden Gattungen: *Acetobacter, Acetobacterium, Acinetobacter, Citrobacter, Enterobacter, Enterococcus, Escherichia, Helicobacter, Klebsiella, Proteus, Providencia, Pseudomonas, Salmonella, Serratia, Staphylococcus, Streptococcus, Actinobacillus, Neisseria, Mannheima, Pasteurella, Candida, Aspergillus, Cryptococcus Trichosporon, Malassezia,* und *Mycobacterium.*

8. Assoziation zu deren Verwendung nach einem der vorstehenden Ansprüche, **dadurch gekennzeichnet, dass** das antimikrobielle Mittel Amoxicillin ist.

9. Assoziation zu deren Verwendung nach einem der vorstehenden Ansprüche, **dadurch gekennzeichnet, dass** das antimikrobielle Mittel eine Amoxicillin-/Clavulansäure-Mischung ist.

10. Assoziation zu deren Verwendung nach einem der vorstehenden Ansprüche, **dadurch gekennzeichnet, dass** das antimikrobielle Mittel Sertaconazol ist.

## Claims

1. Combination of an antimicrobial agent and a compound of formula I for use in treating a microbial infection, the compound having the following formula I:

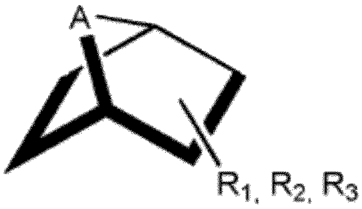

(I)

wherein $R_1$, $R_2$, $R_3$ are each, independently of one another, H, OH, a $(C_1-C_6)$alkyl, $(C_2-C_6)$alkenyl, $(C_1-C_6)$haloalkyl, $(C_1-C_6)$alkoxy, $(C_2-C_6)$alkenoxy, $(C_1-C_6)$haloalkoxy, -O-CO-$(C_1-C_6)$alkyl group;
A is a heteroatom or a -(Het)-R' or R-(Het)-R' group where R, R', are each, independently of one another, a $(C_1-C_4)$alkanediyl group optionally substituted by $C_1$-$C_4$ alkyl radicals and Het is a heteroatom.
in a compound of formula (I): antimicrobial agent mass ratio, ranging from 8:1 to 1:10, in that the antimicrobial agent is not a terpenoid or a phenylpropanoid
and in that the microbial infection is induced in particular by a strain A pathogen and in that on this strain A, the concentration of compound (I) meets the following equation:

$$[C] < [MIC] / x$$

where [C] is the concentration according to the invention of compound (I) to be used on the strain A
[MIC] is the MIC measured for the compound (I), alone, on this strain A x is higher than or equal to 100, advantageously to 1,000, more advantageously x is between 2,000 and 10,000, or even higher than 50,000,

in that said compound of formula (I) is suitable for systemic administration.

2. Combination for its use according to claim 1, **characterized in that** in formula (I), $R_2$ and $R_3$ are each H, $R_1$ is a $(C_1-C_6)$alkyl group, and A is the heteroatom O or an R-O-R' or -O-R' where R, R' group, each represent, independently of one another, a $(C_1-C_2)$alkanediyl group optionally substituted by $C_1$-$C_4$ alkyl radicals.

3. Combination for its use according to claim 1 or 2, **characterized in that** the compound of formula (I) is cineol.

4. Combination for its use according to one of the preceding claims, **characterized in that** said antimicrobial is an antibiotic, in particular an antibiotic chosen from: peptidoglycan synthesis inhibiting antibiotics, nucleic acid synthesis inhibiting antibiotics, folate synthesis inhibiting antibiotics, mycolic acid synthesis inhibiting antibiotics, any of their pharmaceutically acceptable salts, and any of their combinations.

5. Combination for its use according to any of the preceding claims, **characterized in that** the antimicrobial is an antifungal, in particular an antifungal chosen from: polyenes, azoles, allylamines, thiocarbamates, echinocandins, griseofulvin, and fluorocytosine.

6. Combination for its use according to one of the preceding claims wherein the compound of formula (I): antimicrobial agent mass ratio ranges from 4:1 to 1:10, advantageously from 1:1 to 1:10, more advantageously from 1:1 to 1:5.

7. Combination for its use according to one of the preceding claims, **characterized in that** the microbial infection is

induced by a pathogen chosen from the following potentially pathogenic genera: *Acetobacter, Acetobacterium, Acinetobacter, Citrobacter, Enterobacter, Enterococcus, Escherichia, Helicobacter, Klebsiella, Proteus, Providencia, Pseudomonas, Salmonella, Serratia, Staphylococcus, Streptococcus, Actinobacillus, Neisseria, Mannheima, Pasteurella, Candida, Aspergillus, Cryptococcus Trichosporon, Malassezia,* and *Mycobacterium.*

**8.** Combination for its use according to one of the preceding claims, **characterized in that** the antimicrobial is amoxicillin.

**9.** Combination for its use according to one of the preceding claims, **characterized in that** the antimicrobial is an amoxicillin/clavulanic acid mixture.

**10.** Combination for its use according to one of the preceding claims, **characterized in that** the antimicrobial is sertaconazole.

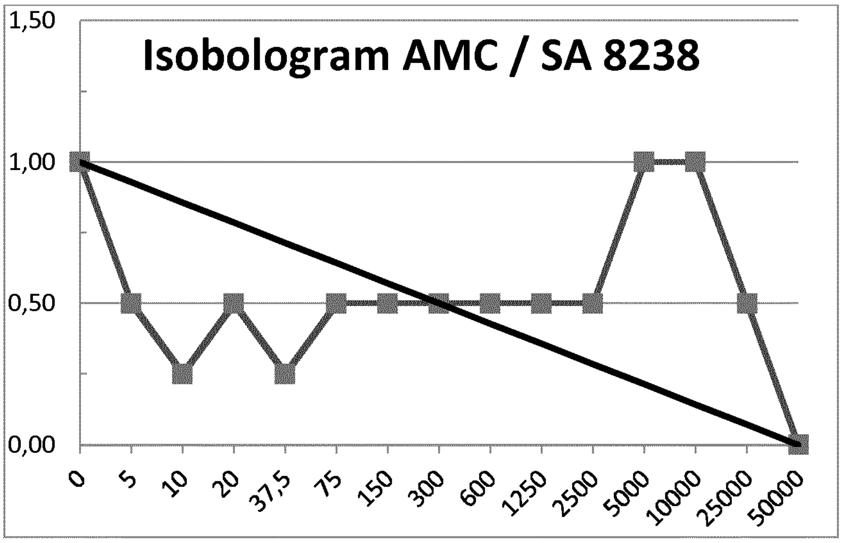
Isobologram AMC / SA 8238
1,50
1,00
0,50
0,00
0
5
10
20
37,5
75
150
300
600
1250
2500
5000
10000
25000
50000

Fig. 1

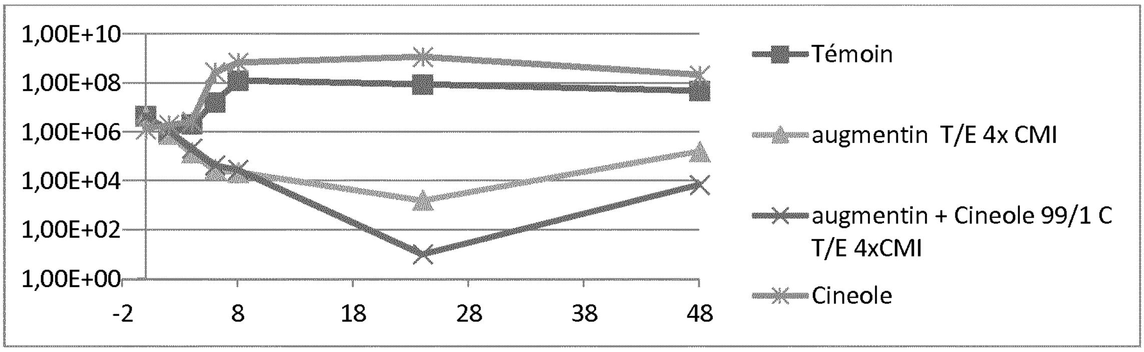
1,00E+10
1,00E+08
1,00E+06
1,00E+04
1,00E+02
1,00E+00
-2
8
18
28
38
48
Témoin
augmentin T/E 4x CMI
augmentin + Cineole 99/1 C T/E 4xCMI
Cineole

Fig 2

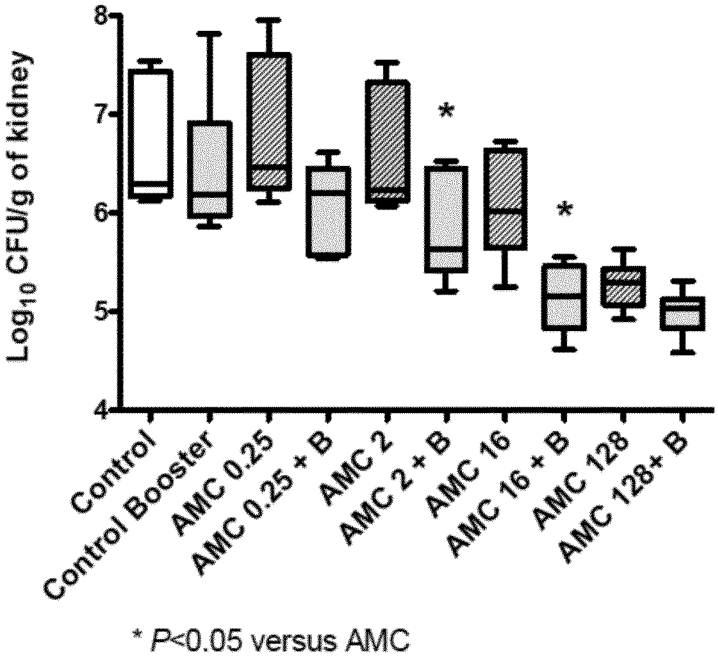
8
7
6
5
4
Log10 CFU/g of kidney
*
*
Control
Control Booster
AMC 0.25
AMC 0.25 + B
AMC 2
AMC 2 + B
AMC 16
AMC 16 + B
AMC 128
AMC 128+ B
* P<0.05 versus AMC

Fig. 3

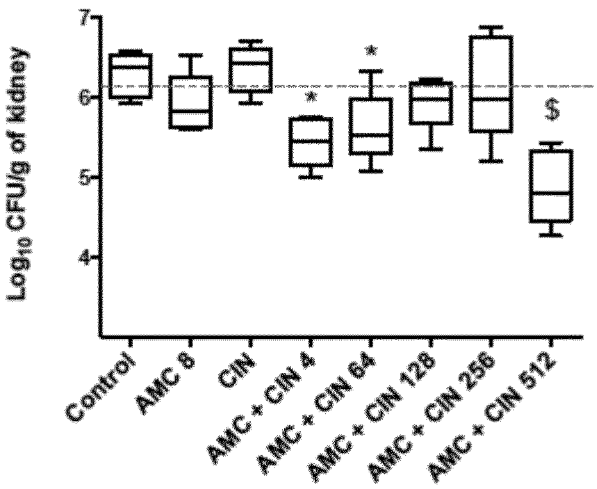
7
6
5
4
Log10 CFU/g of kidney
*
*
$
Control
AMC 8
CIN
AMC + CIN 4
AMC + CIN 64
AMC + CIN 128
AMC + CIN 256
AMC + CIN 512

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9966796 A
- WO 2006120567 A
- DE 19631037
- WO 2009043987 A
- DE 19631037 B


### Littérature non-brevet citée dans la description


- **MARINAS.** *Biointerfacae,* 2012, vol. 2 (1), 271-276
- **HENDRY.** *Journal of Antimicrobial Chemotherapy,* 2009, vol. 64, 1219-1225